(19) **Europäisches Patentamt**
**European Patent Office**
**Office européen des brevets**

(11) **EP 4 096 784 B1**

(12) **EUROPEAN PATENT SPECIFICATION**

(45) Date of publication and mention
of the grant of the patent:
**02.07.2025 Bulletin 2025/27**

(51) International Patent Classification (IPC):
**G01N 33/68** (2006.01)

(21) Application number: **21747785.0**

(52) Cooperative Patent Classification (CPC):
**G01N 33/6893; G01N 2800/347; G01N 2800/52**

(22) Date of filing: **28.01.2021**

(86) International application number:
**PCT/US2021/015391**

(87) International publication number:
**WO 2021/154922 (05.08.2021 Gazette 2021/31)**

(54) **BIOMARKER, METHODS, AND COMPOSITIONS THEREOF FOR EVALUATION OR MANAGEMENT OF KIDNEY FUNCTION OR DIAGNOSING OR AID IN DIAGNOSING KIDNEY DYSFUNCTION OR KIDNEY DISEASE**

BIOMARKER, VERFAHREN UND ZUSAMMENSETZUNGEN DAVON ZUR BEURTEILUNG ODER VERWALTUNG DER NIERENFUNKTION ODER ZUR DIAGNOSE ODER ALS HILFSMITTEL BEI DER DIAGNOSE VON NIERENFUNKTIONSSTÖRUNGEN ODER NIERENERKRANKUNGEN

BIOMARQUEUR, PROCÉDÉS ET COMPOSITIONS ASSOCIÉS POUR L'ÉVALUATION OU LA GESTION DE LA FONCTION RÉNALE OU LE DIAGNOSTIC OU L'AIDE AU DIAGNOSTIC D'UN DYSFONCTIONNEMENT RÉNAL OU D'UNE MALADIE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2020 US 202062968637 P**

(43) Date of publication of application:
**07.12.2022 Bulletin 2022/49**

(73) Proprietor: **Sequela, Inc.**
**Pewee Valley, KY 40056 (US)**

(72) Inventors:
• **COUGHLIN, Richard, T.**
**Falmouth, ME 04105 (US)**
• **CARRITHERS, Stephen, L.**
**Pewee Valley, KY 40056 (US)**
• **CARRITHERS, Aaron, L.**
**Pewee Valley, KY 40056 (US)**
• **CARRITHERS, Brennan, M.**
**Pewee Valley, KY 40056 (US)**

(74) Representative: **August Debouzy**
**7, rue de Téhéran**
**75008 Paris (FR)**

(56) References cited:
**WO-A1-01/25266**          **US-A1- 2008 221 022**
**US-A1- 2008 221 022**

• **QIAN XUN ET AL: "The rat kidney contains high levels of prouroguanylin (the uroguanylin precursor) but does not express GC-C (the enteric uroguanylin receptor)", AMERICAN JOURNAL OF PHYSIOLOGY: RENAL PHYSIOLOGY, vol. 300, no. 2, 1 February 2011 (2011-02-01), United States, pages F561 - F573, XP093107499, ISSN: 1931-857X, DOI: 10.1152/ ajprenal.00282.2010**
• **GERHARD KAAR ET AL: "Proguanylin and prouroguanylin Assay evaluation and clinical analyte characterization", CLINICA CHIMICA ACTA, ELSEVIER BV, AMSTERDAM, NL, vol. 412, no. 23, 15 August 2011 (2011-08-15), pages 2277 - 2283, XP028309204, ISSN: 0009-8981, [retrieved on 20110824], DOI: 10.1016/J.CCA.2011.08.016**

EP 4 096 784 B1

- **ALEKSANDRA SINDIC ET AL: "Mechanisms of actions of guanylin peptides in the kidney", PFLÜGERS ARCHIV - EUROPEAN JOURNAL OF PHYSIOLOGY, SPRINGER, BERLIN, DE, vol. 450, no. 5, 1 August 2005 (2005-08-01), pages 283 - 291, XP019344041, ISSN: 1432-2013, DOI: 10.1007/S00424-005-1464-9**

**Description**

**FIELD OF THE INVENTION**

The present invention relates to the field of medical diagnostic assays and subsequent treatment, and more particularly, to methods of and kits for monitoring kidney function or diagnosing kidney dysfunction or kidney disease in a patient. Specifically, the invention relates to immunoassay detection of a novel biomarker in biological fluid samples, prouroguanylin (proUGN).

**BACKGROUND OF THE INVENTION**

The following background discussion is not admitted to report or comprise prior art to the present invention, but serves to aid the reader in understanding the invention.

The renal system is responsible for essential bodily functions that, if compromised, result in severe systemic pathology. Namely, these functions include but are not limited to water and salt excretion, acid-base balance, electrolyte regulation, blood volume and pressure maintenance, toxin removal, vitamin D metabolism, and production of erythropoietin. Elimination or decline in these functions from injury or disease is known to give significant morbidity/comorbidity and mortality. Renal pathology can be classified on the basis of severity and duration. As such, kidney injury and/or disease can be described as "acute" or "chronic". Each renal pathology is discussed in detail in Robbins Basic Pathology 2018, 10th Ed, Elsevier, Illinois,. "Acute kidney injury refers to abrupt onset in renal dysfunction characterized by an acute increase in serum creatinine often associated with oliguria or anuria (decreased or no urine flow). It can result from glomerular injury (such as rapidly progressive glomerulonephritis), interstitial injury, vascular injury (such as thrombotic microangiopathy), or acute tubular epithelial cell injury." Furthermore, "Chronic kidney disease results from progressive scarring in the kidney of any cause. It is characterized by various metabolic and electrolyte abnormalities such as hyperphosphatemia, dyslipidemia, and metabolic acidosis. However, it is often asymptomatic until the most advanced stages, when symptoms or uremia develop." Kidney dysfunction can progress, and result in, what is currently referred to as end-stage renal disease (ESRD), defined as, "Irreversible loss of renal function requiring dialysis or transplantation typically due to severe progressive scarring in the kidney from any cause."

Chronic kidney disease (CKD) is generally an asymptomatic condition, except in its most advanced state, and thus currently requires blood and/or urine tests to make the diagnosis. In an effort to provide state-of-the-art clinical guidance on the evaluation, management and treatment for all patients with CKD and ensure clarity in definition thereof, Kidney Disease Improving Global Outcomes (KDIGO), Kidney International Suppl, 3, 5-14, 2013, and Kidney Disease Outcomes Quality Initiative (KDOQI™), American J. Kidney Dis., 63, 713-735, 2014, reports CKD as:

1. Kidney damage present for at least 3 months, as defined by structural or functional abnormalities of the kidney, with or without decrease in glomerular filtration rate (GFR), manifest by *either*:

- Pathological abnormalities; or
- Markers of kidney damage, including abnormalities in the composition of the blood or urine, or abnormalities in imaging tests (most often based on increased albuminuria, i.e., urinary albumin/creatinine ratio [UACR] $\geq$30mg/g); and/or

2. Glomerular filtration rate (GFR) <60 mL/min/1.73m$^2$ present for at least 3 months.

Within this framework, KDIGO classified or KDOQI™ defined individuals with CKD into five stages defined by the level of GFR, with higher stages representing lower GFR levels, categorized as follows:

Stage 1: Kidney damage with GFR $\geq$90 mL/min/1.73m$^2$
Stage 2: Kidney damage with GFR 60-89 mL/min/1.73m$^2$
Stage 3: GFR 30-59 mL/min/1.73m$^2$
Stage 4: GFR 15-29 mL/min/1.73m$^2$
Stage 5: GFR <15 mL/min/1.73m$^2$ or kidney failure treated by dialysis or transplantation

Furthermore, KDIGO proposes classification and risk stratification on the basis of cause, GFR category, and albuminuria category. "Cause" of CKD is assigned on the basis of presence or absence of systemic disease and the location within the kidney of observed or presumed pathologic-anatomic findings. Inclusion of "cause" in determining stage of kidney dysfunction was on the basis of prognostic ability and role in cause-specific treatment selection. Classifications for stratifying CKD patients on "GFR category" are assigned as follows:

**GFR categories in CKD**

| GFR category | GFR (ml/min/1.73 m$^2$) | Terms |
|---|---|---|
| G1 | ≥90 | Normal or high |
| G2 | 60–89 | Mildly decreased* |
| G3a | 45–59 | Mildly to moderately decreased |
| G3b | 30–44 | Moderately to severely decreased |
| G4 | 15–29 | Severely decreased |
| G5 | <15 | Kidney failure |

Abbreviations: CKD, chronic kidney disease; GFR, glomerular filtration rate.
*Relative to young adult level
In the absence of evidence of kidney damage, neither GFR category G1 nor G2 fulfill the criteria for CKD.

Inclusion of "GFR category" in determining stage of kidney dysfunction was determined based on its wide acceptance as the best overall index of kidney function, as a reduction in GFR is known to suggest kidney function decline and structural damage. Inclusion of "albuminuria" in determining stage of kidney dysfunction was on the basis of its known ability to reflect disease severity and strong association with progression of kidney dysfunction. "Albuminuria category" are assigned as follows:

**Albuminuria categories in CKD**

| Category | AER (mg/24 hours) | ACR (approximate equivalent) | | Terms |
|---|---|---|---|---|
| | | (mg/mmol) | (mg/g) | |
| A1 | <30 | <3 | <30 | Normal to mildly increased |
| A2 | 30–300 | 3–30 | 30–300 | Moderately increased* |
| A3 | >300 | >30 | >300 | Severely increased** |

Abbreviations: AER, albumin excretion rate; ACR, albumin-to-creatinine ratio; CKD, chronic kidney disease.
*Relative to young adult level.
**Including nephrotic syndrome (albumin excretion usually >2200 mg/24 hours [ACR >2220 mg/g; >220 mg/mmol]).

This recommended system of classification of CKD that is determined on the basis of "Cause", "GFR", and "Albuminuria" is referred to as CGA, respectively. Clinical utilization thereof, comprise indicate need for specialist referral, evaluate and manage disease, discern disease prognosis, and guide specific treatment and/or intervention choice.

Although this classification system of kidney disease addresses the need in clinical practice to acknowledge multiple dimensions and variables by which individual patients are assessed, it does not conform to the International Classification of Disease (ICD) maintained by the World Health Organization (WHO; www.who.int/icd) or ICD-10-CM, maintained by the National Center for Health Statistics (NCHS), Centers of Disease Control and Prevention (CDC) (www.cdc.gov/nchs/icd/icd10cm).

The current standard method for diagnosing "chronic kidney disease", the term CKD as used herein are defined in part in terms of changes in serum creatinine from a baseline value present for at least 3 months. Most definitions of CKD have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{Urine\ Concentration \times Urine\ Flow}{Plasma\ Concentration}$$

By normalizing the GFR to the body surface area, a GFR of approximately 75-100 ml/min per 1.73m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (C[Cr]) can be calculated if values for creatinine's urine concentration (U[Cr]), urine flow rate (V), and creatinine's plasma concentration (P[Cr]) are known. Since the product of urine concentration and urine flow rate yields creatinine's excretion rate, creatinine clearance is also said to be its excretion rate (U[Cr] x V) divided by its plasma concentration. This is commonly represented mathematically as:

$$C[Cr] = \frac{U[Cr] \text{ x } V}{P[Cr]}$$

Commonly, a 24-hour urine collection is undertaken from empty-bladder one morning to the contents of the bladder the following morning, with a comparative blood test then taken:

$$C[Cr] = \frac{U[Cr] \text{x } (24hr \ volume)}{P[Cr] \text{x } (24 \text{ x } 60 \ minutes)}$$

To allow comparison of results between people of different sizes, the C[Cr] is often corrected for the body surface area (BSA) and expressed and compared to the average sized man as ml/min/1.73m$^2$. While most adults have a BSA that approaches 1.7 (1.6 - 1.9), extremely obese or slim patients should have their C[Cr] corrected for their actual BSA:

$$C[Cr] - corrected = \frac{C[Cr] \text{x } (1.73)}{BSA}$$

The accuracy of a creatinine clearance measurement (even when collection is complete) is limited because as glomerular filtration rate (GFR) falls, creatinine secretion is increased, and thus the rise in serum creatinine is less. Thus, creatinine excretion is much greater than the filtered load, resulting in a potentially large overestimation of the GFR (as much as a two-fold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of kidney disease. Nonetheless, the degree to which serum creatinine changes from baseline will reflect the change in GFR. Serum creatinine is readily and easily measured and it is specific for renal function.

The standard of care laboratory tests currently used to screen and diagnose kidney dysfunction are based on the serum creatinine, which utilizes an equation-based scale to generate an estimated glomerular filtration rate (eGFR), and the urine albumin-creatinine ratio (UACR) [1-3]. A recent retrospective analysis consisting of 47,104 adults demonstrated that a single assessment eGFR for detecting CKD in patients with a first-time low value (<60 ml/min/1.73m$^2$) between 45-59 ml/min/1.73m$^2$ had a sensitivity of only 20-28%, range depending on age, and PPV (positive predictive value) of 62% [4]. Based on recent microalbuminuria data in CKD patients from Kidney Disease Improving Global Guidelines Outcomes (KDIGO [5]) and the National Health and Nutrition Examination Survey (NHANES), the estimated UACR single assessment sensitivity for identifying CKD was 24% and a PPV of 15% [5-7]. The PPV for both eGFR and UACR are too low to confirm that a patient has CKD on a single assessment [2, 5, 8-13]. Henceforth, current guidelines require that the gold standard laboratory assays - eGFR and UACR - adhere to a temporal criterion in which reported results must remain consistently abnormal over at least a 3-month period of time to distinguish "chronic" kidney dysfunction from "acute" or "subacute", which are indiscernible by current measures [5, 13-15].

The International Society of Nephrology (ISN) estimates that over 850 million people worldwide have some form of kidney dysfunction, and chronic kidney diseases (CKD) are by far the most common, with an estimated global prevalence of 10.4% in men and 11.8% in females [16, 17]. Kidney diseases are largely considered "silent diseases", as most often there are no apparent symptoms in the early stages [16, 17]. As such, diagnosis is considered difficult, and many patients with the disease are commonly unaware of their condition [3, 5, 15, 17-20]. The National Health and Nutrition Examination Survey (NHANES [21]) estimates that adults who were clinically confirmed by laboratory measurements to have CKD, the percentage aware of their disease was <10% for Stages 1-3 and <60% for Stage 4 [1-3, 13, 19, 20]. Additionally, the rate of clinical diagnosis of US adults with CKD is only 26.5% (95% confidence interval, 17.9 to 35.1), or in other words, >70% of patients with CKD are currently undiagnosed [39].

Current medical practice standard of care screening guidelines for kidney disease provided by the American Academy of Family Physicians (AAFP) note, "Because this disease can silently progress to advanced stages, early detection is critical for initiating timely interventions." The insidious nature and long asymptomatic phase of CKD has been associated with a high and rising prevalence and numerous health conditions such as increased risk of mortality, cardiovascular disease, fractures, bone loss, infections, cognitive impairment, and fragility. Asymptomatic diagnostic screening, early diagnosis, and a multidisciplinary management approach implementing early interventions and treatments, providing education, and planning for advanced renal stage disease is an effective means to alter the course of early-stage CKD, deter the progression of late-stage, and reduce associated complications and adverse health outcomes.

Accordingly, screening at risk, asymptomatic individuals is recommended by the AAFP, "Multiple guidelines recommend at least annual screening with serum creatinine, UACR, and urinalysis for patients with risk factors, particularly hypertension, diabetes mellitus, and a history of cardiovascular disease." Yet, abnormal screening test results still must adhere to the aforementioned temporal criterion required for a CKD diagnosis, as a reliable single-assessment approach, or method, that tests for kidney dysfunction or disease is currently not practical for use as a standard of practice for medical care by the AAFP, or any other established internationally recognized kidney society.

Rationale to improve diagnostic efficacy on single-assessment for kidney disease is validated by the significant proportion of patients who are lost to follow-up after the initial screening test(s), and hence cannot be clinically diagnosed. Responsively, critical milestones that focus on preventing, diagnosing, and treating kidney disease are determined by Healthy People 2030, specifically, Goal CKD-03: Increase the proportion of high-risk patients who have a follow-up evaluation of their kidney function 3 months after renal damage or low renal function is detected (i.e., only "10.5% of Medicare beneficiaries who incurred AKI had a follow-up evaluation of their kidney function within 3 months post discharge").

Recognizing the factors associated with CKD initiation and progression enables high-risk patients to be identified and treated more appropriately and earlier in the course of disease. Specifically, Healthy People 2030 Goal CKD-02: Increase the proportion of persons with chronic kidney disease (CKD) who know they have impaired renal function. The National Institute of Diabetes and Digestive and Kidney Diseases (NIDDK) at National Institutes of Health (NIH) has listed the following aims to help meet the Healthy People 2030 Goal [20, 22, 23]:

Aim II.A. *"Identification of surrogate markers in the plasma or serum that correlate with acute or chronic kidney disease..."*

Aim III.K. *"Development of new non-invasive methods for measuring kidney function: 1. Reliable, non-invasive, non-radioactive methods of measuring glomerular filtration rate (GFR); 2. Identification of serum factors released by damaged kidney cells; 3. Characterization of changes in kidney hormonal function in kidney disease at various stages of severity; and 4. Development of new biomarkers for early detection of kidney dysfunction."*

New drugs are currently being evaluated that may improve kidney function if implemented early in the clinical course [24, 25], however there are no authentic and valid innovations in biomarkers for CKD that can assess renal function and dysfunction early in the stage of disease, improve diagnostic efficiency of CKD, or help determine efficacy of the new medications following implementation. Thus, as stated by National Kidney Disease Educational Program (NKDEP), which was established by the NIDDK, "The benefits of earlier intervention of kidney disease are numerous and important to research; early detection of CKD must improve" [20].

Prouroguanylin (proUGN) is produced and secreted from intestinal cells, renal tubular cells, and other endocrine/exocrine cells that help manage salt and water transport locally (within a specific tissue) and within the body [26-28]. Sindić and Schlatter (2005) describe guanylin peptides as natriuretic peptides and more particularly describe their mechanisms of action in the kidney (Sindić A, Schlatter E. Pflugers Arch. 2005 Aug;450(5):283-91.). US 2008/0221022 relates to methods for treating or diagnosing fluid and/or salt imbalance using prouroguanylin and/or active derivatives thereof.

The prohormone biomarker proUGN is released apically into the lumen and basolaterally into the vascular space and bloodstream [28-32]. This precedent pertains not only to intestinal enteroendocrine and enterochromaffin cells [33-35], but also to specific epithelial cells lining the nephron [36-40], pancreas [41, 42], and hypothalamic neurosecretory cells [43, 44]. Once into circulation, the prohormone is then cut near the site of the kidney receptor lining within the lumen of the nephron into its 16-amino acid bioactive moiety, termed uroguanylin (UGN) [45-47]. Kaar et al (2011) performed an assay evaluation of a prouroguanylin commercially available ELISA and characterized prouroguanylin in terms of in vitro analyte stabilities, biological variability, and reference values, even though its role in chronic heart failure is not fully established (Kaar G, Dieplinger B, Gabriel C, Haltmayer M, Mueller T. Proguanylin and prouroguanylin--assay evaluation and clinical analyte characterization. Clin Chim Acta. 2011 Nov 20;412(23-24):2277-83).

To date, the intact proUGN prohormone molecule has not been found to bind directly to any receptor in the body or have any pharmacologic and/or functional importance, other than being the transfer message (i.e., carrier) of the bioactive and mature UGN peptide. UGN binds to the guanylate cyclase C (GC-C) receptor (localized within the lumen of specific regions of the nephron) and initiates an intracellular cyclic guanosine monophosphate (cGMP)-dependent signaling cascade [26, 28, 29, 40, 48-52]. Although production of and presence of intraluminal, circulating, and urinary proUGN is derived from many sources, including basolateral secretion from specific renal tubule cells into the bloodstream, the accumulation of proUGN in the blood or any other biological fluid requires, in part, a failure of the kidney to process the proUGN prohormone to its active UGN form and/or filtration of proUGN by the kidney, a novel discovery critical to the methods and development of the invention described herein.

## SUMMARY OF THE INVENTION

The invention is set out in the appended set of claims.

The present invention provides methods and compositions thereof for use in the diagnosis, or providing guidance of clinical decision-making regarding management strategies of renal dysfunction, including renal disease. More specifically, the invention provides a simple blood or other bodily fluid test with high sensitivity and specificity for proUGN or fragments or post-translational modifications thereof which provides the means for an early diagnosis and staging of renal dysfunction and/or renal disease, specifically using one or multiple assessments, in which the interpretation of the measurement would indicate a stage-specific clinical management treatment protocol, and monitor that renal dysfunction or disease status in response to the indicated treatment(s) thereof.

More particularly the invention relates to a method of

(i) diagnosing a renal dysfunction or disease and/or renal complications of a disease in a subject, or
(ii) diagnosing or monitoring kidney function in a subject, or
(iii) diagnosing kidney disease or renal complications prior to the onset of kidney degeneration in a subject, or
(iv) predicting or monitoring the risk of an adverse event in a subject with kidney disease wherein said adverse event is selected from the group comprising worsening of kidney dysfunction including kidney failure, loss of kidney function, development of related comorbid condition, end-stage kidney disease, or death, or
(v) predicting or monitoring the success of a therapy or intervention in a subject with kidney disease, the method comprising:

(a) measuring a level or amount of prouroguanylin (proUGN) in a biological sample,
(b) comparing the measurement to a control reference range or threshold value,
(c) determining whether the measurement of proUGN is above the control reference range or threshold value.

Methods are also provided for evaluating the efficacy of treatments using the described reagents.

The present invention is also directed as a kit for:

(i) diagnosing a renal dysfunction or disease and/or renal complications of a disease in a subject, or
(ii) diagnosing or monitoring kidney function in a subject, or
(iii) diagnosing kidney disease or renal complications prior to the onset of kidney degeneration in a subject, or
(iv) predicting or monitoring the risk of an adverse event in a subject with kidney disease wherein said adverse event is selected from the group comprising worsening of kidney dysfunction including kidney failure, loss of kidney function, development of related comorbid condition, end-stage kidney disease, or death, or
(v) predicting or monitoring the success of a therapy or intervention in a subject with kidney disease,

encompassing one or more containers, comprising:

(a) a substrate having a surface and a first proUGN antibody adhered to the surface;
(b) a second proUGN antibody comprising a detectable marker;
wherein at least one of the first and second proUGN antibodies are monoclonal antibodies which specifically binds to human proUGN, or a fragment or post-translational modification thereof, wherein the human proUGN, or the fragment or post-translational modification thereof is secreted from a human kidney cell; and
(c) instructions for performing one or more of the diagnostic and/or prognostic correlations recited in any one of (i) to (v).

In an embodiment of said kit, the first and second proUGN antibodies are monoclonal antibodies.

In another embodiment of said kit, the first and second proUGN antibodies are capable of binding proUGN having the sequence as set forth in SEQ ID NO:1.

In a further embodiment of said kit, the disease is chronic kidney disease (CKD).

The method can be used for stratifying a patient suffering from undiagnosed CKD into "Early CKD", or one of Stages 1, 2, or 3 from those having a more severe form of the disease, or "Late CKD", such as Stages 4 or 5, wherein Stage 5 is also defined as end-stage renal disease ("ESRD") [53]; and differentiating those groups from a population whose kidney function is determined to be within normal limits. This is accomplished by determining the level of the biomarker proUGN measured in said biological fluid sample from the patient (i.e., biological fluid sample including but not limited to serum, plasma, blood, urine, saliva, lung lavage, nasal lavage, cerebrospinal fluid, gastrointestinal fluid, amniotic fluid, exosomes thereof, etc.) and comparing the level of proUGN in the sample to that of a control reference range or a threshold value, wherein increased proUGN levels elevated above a normal control reference range but below the lower limit of a lower threshold value and creatinine levels within the range of control values indicate Stage 1 or 2 CKD; wherein an increased level of proUGN exceeding the lower limit of a predetermined range of control values that is defined by the lower threshold indicates that the patient suffers from CKD Stage 3, 4, or 5; wherein an increased level of proUGN exceeding the upper limit of an intermediate threshold value indicates "Late CKD" or CKD Stage 4 or 5; wherein proUGN levels elevated above the upper limit of a predetermined range of control values defined by an upper threshold indicates ESRD, or CKD Stage 5.

The method can be used for determining whether an individual has renal dysfunction comprising determining the level of proUGN in a biological fluid sample from the patient by contacting the sample with a solid-state device comprising a substrate having an activated surface onto which is applied an antibody to proUGN to discreet areas of the activated surface and comparing the level of proUGN in the sample to a control value threshold of proUGN, wherein an increase in proUGN compared to the control value indicates the patient has renal dysfunction or renal disease.

The method can be used for determining the efficacy of a treatment for CKD, comprising stratifying the stage of a patient suffering from CKD according to the method of the fourth aspect of the present invention before and after treatment, wherein a decrease in proUGN level or reduction in renal disease stage indicates treatment success.

The method is based on the use of the biomarker, proUGN, and/or fragments or post-translational modifications thereof, associated with normal kidney function that advances the diagnostic efficiency of kidney disease alone or with other biomarkers (i.e., proUGN levels appear to be complementary, in part, to filtration-dependent markers). Additionally, methods thereof provide a diagnostic or screening test that is simple to use, stratifies patients by level or risk, determines severity or stage, and determines treatment based on the determined level or risk.

Overall, utilization of methods thereof provides means of clinical decision-making regarding treatment and management.

## BREIF DESCRIPTION OF DRAWINGS

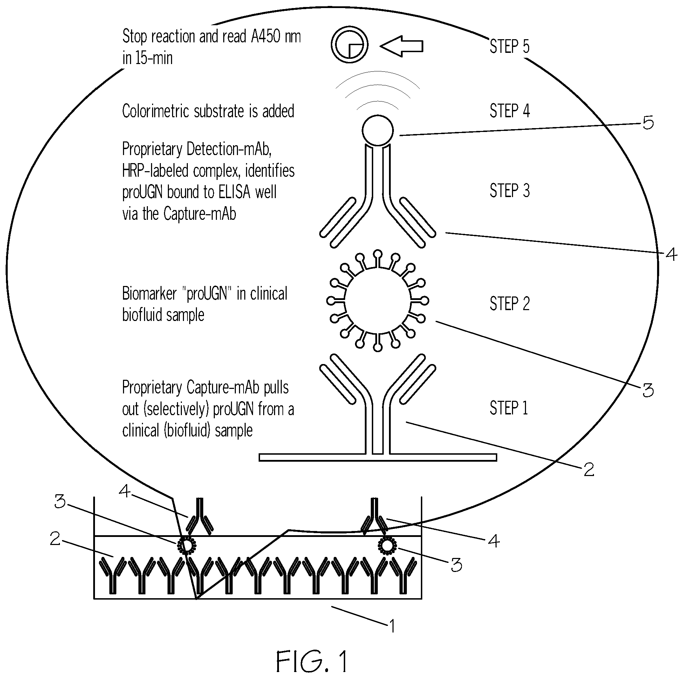

**Figure 1** shows a diagram of the prouroguanylin (proUGN) sandwich immunoassay detection system, depicting one particular embodiment of the invention of the present disclosure described herein. *(1)* Inner surface of a representative well in a multi-well plate. *(2)* Capture monoclonal antibodies (mAb) specific to the biomarker proUGN adhered and coated on the inner surface of the well. *(3)* Biomarker proUGN in a biological/clinical sample such as serum, plasma, blood, urine, saliva, lung or nasal lavage, cerebrospinal fluid, gastrointestinal fluid, amniotic fluid, and exosomes thereof, etc. *(4)* Detection mAb's conjugated to horseradish peroxidase (HRP); the complex is selective to proUGN. *(5)* The HRP is conjugated to the detection mAb's and is activated by the addition of tetramethylbenzidine (TMB). The color development prompted by the addition of TMB is quenched by the addition of STOP-acid solution (a mixture of hydrochloric and sulfuric acid). This particular exemplary embodiment is an enzyme linked chemiluminescent or immunofluorescent sandwich assay that involves five steps (shown in Example 3).

**Figure 2** shows serum proUGN levels in patients with renal disease. Groups are stratified by clinically diagnosed renal disease stage and compared against a healthy control donor sample population.

**Figure 3** shows a receiver-operator curve (ROC) analysis to determine the specificity (96.9%) and sensitivity (98.5%) for the invention to discriminate between a control group and all CKD patients.

**Figure 4** shows a receiver-operator curve (ROC) analysis to determine the specificity (88.9%) and sensitivity (84.7%) for the invention to discriminate between "Late CKD" and "Control + Early CKD" patient groups, as defined by KDIGO.

**Figure 5** shows a receiver-operator curve (ROC) analysis to determine the specificity (96.2%) and sensitivity (92.9%) for the invention to discriminate between Stage 5, or "ESRD", as defined by KDIGO, versus "Control + Early CKD + Stage 4 CKD" patient groups.

## DETAILED DISCUSSION OF THE INVENTION

The present invention relates to methods and compositions for diagnosis, monitoring, classifying, staging, and determination of treatment regimens in subjects suffering or at risk of suffering from reduced renal function, state of kidney disease chronicity, renal injury or renal failure by determining the level of proUGN in a biological fluid sample from a patient and comparing the level of proUGN with a control reference range or threshold value. An increased level of proUGN in the sample compared to the control indicates that the patient suffers from renal dysfunction. Further, based on the detection of renal dysfunction, the subject can be treated, which is not part of the claimed invention.

For purposes of this document, the following definitions apply:

As used herein, "renal dysfunction" is defined in the context of the present invention as ailments or diseases related to decreased kidney function relative to a healthy patient population. By way of example, renal ailments or diseases may include but are not limited to chronic kidney disease (CKD), acute kidney injury (AKI), diabetic nephropathy, cystic diseases of the kidney, glomerulonephritis, minimal-change disease, focal glomerulosclerosis, IgA nephropathy, amyloidosis, immune complex nephropathies, lupus nephritis, as well as blood vessel pathologies such as nephrosclerosis, malignant hypertension, or thrombotic microangiopathies. Renal dysfunction in the context of the present invention may also include drug-induced kidney injury or graft rejection. Renal dysfunction in the context of the present invention may also be characterized as renal insufficiency. As used herein, "chronic kidney disease" or "CKD" is defined as abnormalities of kidney structure or function present for at least 3-months, with implications for health.

As used herein, the term "subject" as used herein refers to a human or non-human organism. Thus, the methods and compositions described herein are applicable to both human and veterinary disease for clinical, medical, or research purposes. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for potential signs of disease.

As used herein, "control reference range" or "threshold value" is defined in the context of the present invention as the quantification of a specific biomarker that is most commonly measured from a healthy patient population, wherein the "control reference range" include the amounts or levels of proUGN or fragments or post-translational modifications thereof, from a sample that imply a certain state of normal renal function, wherein "threshold value" is defined as the indicated level of proUGN or fragments or post-translational modifications thereof, from a sample in which renal function demonstrates transition to a lower or to a higher state of renal function or stage of renal disease. A control value can be determined by analyzing the sample of a healthy patient or by calculating an average value from a healthy patient population or may be the value understood by the skilled person to be considered normal among healthy patients. The skilled person would understand that the control value is determined by sample analysis of a healthy individual or by reference range of values predetermined as the typical value found in healthy patients, i.e. a normal range of values for subjects without renal disease, by the assay manufacturer or developer.

The term "biological fluid sample" as used herein refers to a sample of bodily fluid used for the purpose of diagnosis, prognosis, classification or evaluation of a subject of interest, such as a patient or transplant donor. The biological fluid sample matrix isolated from the patient is preferably serum, but is not limited in this nature, and may also include plasma, urine, whole blood, saliva, or another similar matrix. The determination of the level of proUGN may be done on one or more samples from the patient. In certain embodiment, such a sample may be used for the purpose of determining the outcome of an ongoing condition, or the effect of a treatment regimen on a condition..

"Diagnosis" or "aid to diagnosis" as used herein refers to methods by which the skilled artisan can estimate and/or determine the probability ("a likelihood") of whether or not a patient is suffering from a given disease or condition, or aid thereof. In the case of the present invention, "diagnosis" includes using the results from an assay, most preferably an immunoassay, for a novel biomarker of kidney dysfunction, optionally together with other clinical characteristics, to arrive at a diagnosis (that is, the occurrence or nonoccurrence) of kidney dysfunction, most preferably the early diagnosis of CKD, for the subject from which a sample was obtained and assayed. That such a diagnosis is "determined" is not meant to imply that the diagnosis is 100% accurate. The skilled clinician uses clinical indicia combined with laboratory test results, including biomarker levels, to arrive at a diagnosis. Thus, a measured biomarker level below or above a predetermined diagnostic threshold value or control reference range indicates a decreased or increased likelihood of the occurrence of disease in the given subject.

In healthy individuals, the levels of proUGN are present at a low maintenance level supporting their role in the regulation of sodium retention. The present inventors have found a rise in proUGN levels in a stepwise fashion significantly above normal levels in individuals with kidney dysfunction and that there is a stepwise increase in proUGN in samples obtained from patients with clinically diagnosed CKD henceforth providing objective information that may be used to identify or stage disease and progression thereof. In the context of the current invention, "Early CKD" is understood to mean any of the first to third stages of CKD as defined by the KDOQI classification; whereas "Late CKD" is understood to mean either fourth or fifth stages of CKD as defined by the KDOQI classification; whereas "ESRD" is defined as the fifth stage of CKD or dependent on the use of dialysis as defined by KDOQI classification. Furthermore, the present invention may be used to diagnose CKD at any given stage significantly more early in the disease course compared to current clinical diagnostic techniques, therefore allowing earlier implementation of stage-specific treatment or management.

The stratification of patients as in one of the stages of CKD is useful to assess whether a patient would benefit from one treatment type compared to another, and also to monitor whether a treatment is successful. Stratification of patients also aids in determining short- and long-term prognosis of the patient, thereby enabling future care requirements.

The method of the current invention identifies renal disease in a patient by comparing the levels of proUGN to a control reference range, wherein guidance of treatment is provided by stratification of renal disease by grade of severity or stage by comparison of proUGN levels to predetermined threshold values, wherein the patient is indicated to begin either "Early CKD" or "Late CKD" clinical management protocol; wherein a diagnosis of ESRD indicates the need for dialysis treatment (as shown in Example 6).

Estimated GFR (eGFR) is inversely correlated to creatinine levels. An increase in creatinine compared to a control value in patients presenting with increased levels of proUGN allow the patients to be stratified as Stage 1 or Stage 2 CKD. Thus, the method of the invention may further compromise the level of creatinine in the sample and comparing the levels with normal ranges suggested by the assay's instructions for use (as shown in Example 5). Patients having increased levels of proUGN compared to a normal control reference range of values and below a predetermined lower threshold and creatinine levels within a range of control values are indicated to have Stage 1 or 2 CKD (as shown in Examples 4 and 5); wherein patients having proUGN levels above the lower limit of control values defined by a predetermined lower threshold value and below a predetermined intermediate threshold value are classed as Stage 3 CKD; wherein patients having increased levels of proUGN compared to an upper limit of an intermediate threshold value may be classed as Stage 4 or 5 CKD and are indicated to be "Late Stage"; where patients having increased levels of proUGN compared to the upper limit of a range of control values defined by a predetermined upper threshold value indicates "ESRD". Thus, the methods of the present invention allow for the stratification of a patient having renal dysfunction as a non-CKD patient or a patient at one of the Stages 1 to 5 of CKD and further indicating a stage-specific treatment protocol. The stratification of the patient may be defined by the preferred clinical indicia, wherein one skilled in the art is aware that it is not meant to be a limiting embodiment but to be exemplary in nature only, which is detailed by the following criteria:

"Non-CKD" - proUGN within a normal control reference range (detailed in Example 4) calculated by the skilled person from a cohort of healthy individuals;

"Stage 1 or 2" - proUGN above the normal control reference range and lower than the lower threshold value, preferably defined by 1.70 ng/mL and creatinine within a range of control values that correlates with eGFR $\geq 60$ mL/min/1.73m$^2$;

"Stage 3" - proUGN above a lower threshold value, preferably 1.70 ng/mL, and below an intermediate threshold value, preferably 2.53 ng/mL;

"Stage 4" - proUGN above an intermediate threshold value, preferably 2.53 ng/mL, and below an upper threshold value, preferably 5.93 ng/mL;

"Stage 5" - proUGN above an upper threshold value, preferably 5.93 ng/mL;

The reference ranges and threshold values for the biomarker proUGN are described in Tables 1 and 2 and are shown in detail in Examples 4 and 5.

Biomarker Assay.

The methods of the present invention may use methods for determining the level of proUGN known in the art, such as enzymatic and/or chemical protein determination or immunological assay-based methods. Immunological assays for determining the level of proUGN can be performed in a variety of formats including sandwich assays (i.e., enzyme linked immunosorbent assay; ELISA), competition assays (i.e., competitive radioimmunoassay; RIA), lateral flow assays, and non-competitive assay formats, to generate a signal that is related to the presence or amount of the biomarker of interest. Suitable assay formats also include chromatographic, mass spectrographic, and protein "blotting" methods. Accordingly, the quantitative measure of prouroguanylin (proUGN) comprises an immunological assay selected from the group consisting of enzyme linked immunosorbent assay (ELISA), lateral flow assay, counting immunoassay (CIA), chemiluminescence immunoassay (CLIA), radioimmunoassay (RIA), immunoradiometric assay (IRMA) and Western blot, preferably ELISA. In an embodiment, the lateral flow assay comprises capturing the proUGN with a capture monoclonal antibody, and binding a detection monoclonal antibody to the proUGN.

Additionally, certain methods and devices, such as biosensors and optical immunoassays, may be employed to determine the presence or level of analytes without the need for a labeled molecule. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. Methods for determining the level of proUGN include contacting the patient sample with antibodies that binds to proUGN and detection binding.

In general, immunoassays are specific binding assays that involve contacting a sample containing or suspected of containing a biomarker of interest with at least one antibody that specifically binds to the biomarker. A signal is then generated indicative of the presence or level of complexes formed by the binding of polypeptides in the sample to the antibody. The signal is then related to the presence or amount of the biomarker in the sample.

While the present application describes an antibody-based binding assay in detail, alternatives to antibodies as binding species or binding moieties in assays are well known in the art. These include receptors for a particular target biomarker, GC-C receptor, aptamers, etc. GC-C receptor is naturally expressed in humans and has an exceptionally high affinity for proUGN. Aptamers are oligonucleic acid or peptide molecules that bind to a specific target molecule. Aptamers are created by selection from large random sequence pools, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities. Another available binding moiety is a non-Ig-scaffold binding protein. In an embodiment, the binding moiety is specific for prouroguanylin (proUGN), or fragments or post-translational modifications thereof. In an embodiment, the binding moiety is an antibody, an antibody fragment, or a non-Ig-scaffold binding protein.

In an embodiment, the binding moiety binds to a region of at least 4 amino acids of full-length prouroguanylin (proUGN), an N-terminal fragment of proUGN, or a C-terminal fragment of proUGN.

The full-length prouroguanylin (proUGN) has the sequence "vyiqyqgfrvqlesmkklsdleaqwapsprlqaqsllpavchh palpqdlqpvcasqeassifktlrtianddcelcvnvactgcl " (SEQ ID NO: 1), the N-terminal fragment of proUGN has the sequence "vyiqyqgfrvqlesmkklsdleaqwapsprlqaqsllpavchhpalpqdlqpvcasqeassifktlrtia" (SEQ ID NO: 2), and the C-terminal fragment of proUGN has the sequence "tianddcelcvnvactgcl" (SEQ ID NO: 3) and the C-terminal fragment of proUGN has the sequence "nddcelcvnvactgcl" (SEQ ID NO: 4).

Preferably, a solid-state device may be used to determine the level of proUGN in the sample isolated from the patient. The solid-state device comprises a substrate having an activated surface on to which is applied antibodies or other polypeptides to proUGN may be immobilized to discreet areas of the activated surface.

A device that may be used in the invention may be prepared by activating the surface of a suitable substrate, and applying an array of antibodies onto discrete sites on the surface. Antibodies or other polypeptides may be immobilized onto a variety of particles or other solid supports, and that solid support immobilized to the device surface. Solid phases that may be used to immobilize specific binding members include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding.

Such assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (i.e., enzymes such as horseradish peroxidase, alkaline phosphatase, etc.) or by a specific binding molecule which itself may be detectable (i.e., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (i.e., containing identical reactive groups) and heterofunctional cross-linkers (i.e., containing non-identical reactive groups). Homobifunctional cross-linkers that couple through amines, sulfhydryls or react non-specifically are available from many commercial sources. Maleimides, alkyl- and arylhalides, alpha-haloacyls and pyridyl disulfides are thiol reactive groups. Maleimides, alkyl- and aryl- halides, and alpha-haloacyls react with sulfhydryls to form thioether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention comprises kits for measurement of proUGN from the given biological fluid sample. The kit comprises reagents for the analysis of at least one test sample which comprise at least one antibody that bind each biomarker being assayed. The kit can also include devices and instructions for performing one or more of the diagnostic and/or prognostic correlations described herein. Preferred kits will comprise an antibody pair for performing a sandwich assay, or a labeled species for performing a competitive assay, for the analyte. In this embodiment, the antibody pair comprises a first antibody conjugated to a solid phase and a second antibody conjugated to a detectable label, wherein each of the first and second antibodies capable of binding proUGN have the sequence as set forth SEQ ID NO: 1. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies or Binding Apparatus.

The term "antibody" as used herein refers to a peptide or polypeptide derived from, modeled after or substantially encoded by an immunoglobulin gene or immunoglobulin genes, or fragments thereof, capable of specifically binding an antigen or epitope. See, i.e., Fundamental Immunology, 3rd Edition, W.E. Paul, ed., Raven Press, N.Y., 1993; Wilson, J.

Immunol. Methods 175, 267-273, 1994; and Yarmush, J. Biochem. Biophys. Methods 25, 85-97, 1992. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., Nature 341, 544-546, 1989), which consists of a VH domain; and (vi) an isolated complementarity determining region (CDR). Single chain antibodies are also included by reference in the term "antibody."

Antibodies used in the immunoassays described herein preferably specifically bind to proUGN or fragments or post-translational modifications thereof. In an embodiment, the antibodies bind to a region of at least 4 amino acids of full-length prouroguanylin (proUGN), an N-terminal fragment of proUGN, or a C-terminal fragment of proUGN.. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is at least 5-fold greater when compared to its affinity for a non-target molecule which does not display the appropriate epitope(s).

The term "epitope" refers to an antigenic determinant capable of specific binding to an antibody. Epitopes usually consist of chemically active surface groupings of molecules such as amino acids or sugar side chains and usually have specific three-dimensional structural characteristics, as well as specific charge characteristics. Conformational and non-conformational epitopes are distinguished in that the binding to the former but not the latter is lost in the presence of denaturing solvents.

Assay Correlations.

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold value involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. Alternatively, in situations where treatment options are less effective and have higher risk, clinicians often need a higher degree of diagnostic certainty. Thus, cost/benefit analysis is often involved in selecting a diagnostic threshold, as well.

Population studies may also be used to select a decision threshold. Receiver Operating Characteristics (ROC) analysis is often used to select a threshold able to best distinguish a "diseased" subpopulation from a "non-diseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity, and FPR is equal to (1 - specificity), the ROC graph is sometimes called the sensitivity versus (1 - specificity) plot. A perfect test will have an area under the ROC curve of 1.0; a random test will have an area of 0.5. A threshold value is selected to be the clinical cut-off line that discerns between the presence or absence of a diseased state within an individual patient and provides an acceptable level of specificity and sensitivity.

In this context, "diseased" is meant to refer to a population having one characteristic (the presence of a disease or condition or the occurrence of some outcome) and "non-diseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Clinical indicia which may be combined with the proUGN biomarker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TEVII Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatinine ratio, and/or a renal failure index calculated as urine sodium / (urine creatinine / plasma creatinine). Other measures of renal function which may be combined in the methods of the present invention are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

The invention is further described with reference to the following non-limiting examples:

Example 1: Stable Expression of Human proUGN in Human Kidney Cell-Line.

An immortalized human kidney cell line stably transfected with a codon-optimized nucleic acid sequence comprising a gene encoding a human target protein and employing a human cell expression vector including but not limited to mammalian transfection vector pcDNA 3.1 that constitutively expresses the target protein can be made by conventional means known to people skilled in the art of molecular biology. Said immortalized human kidney cell line does not derive from a human embryonic cell. In one embodiment, the human embryonic kidney cell selected from 293 cells (HEK293, ATCC CRL-1573) can be transfected with a mammalian transfection vector that includes and comprises the gene encoding human preprouroguanylin (NCBI human gene ID: 2981, GUCA2B) without any tags and containing the necessary and required N-terminal sequence for appropriate intracellular processing, folding, packaging, and secretion of the proUGN. HEK293 cells can be initially cultured in DMEM/F-12 supplemented with 10% FBS in adherent format. HEK293 cells can be seeded at $0.35 \times 10^6$ in a 6-well plate one day prior to transfection to ensure ~70-80% confluency in 24-hrs under standard growth conditions for HEK293 cells. The mammalian transfection vector (~6 ug) can be transfected using standard protocols, antibiotic additives, and growth conditions according to manufacturer's instructions and by methods described by Thomas and Smart, 51(3), p187-200, 2005 and Aydin et al., J. Vis. Exp. 65, e4041, 2012. Successfully transfected cells can be sorted by flow cytometry and identified by Western blot and ELISA for the target protein. Individual clones expressing and secreting the target protein can be selected and seeded into twelve 96-well plates at ½ cell per well, and allowed to grow in culture media until 100% confluency. The cell media / cell supernate can be removed and tested for proUGN by Western blot and ELISA. Positive wells / clones can be collected and each clone can be seeded again to twelve 96-well plates at ½ cell per well, and grown to confluency. Confirmation of proUGN expression and secretion into the cellular media / cell supernate is confirmed through Western blot analyses and ELISA. A third round of selection for high and efficient proUGN-expressing HEK293 cells can be performed and evaluated by Western blot analyses and proUGN ELISA, and selected. The clone(s) with the highest expression of proUGN can be transitioned to serum-free media in a stepwise fashion decreasing the FBS supplement by 50% every 7-14 days until which time the HEK293 cells are grown and sustained in serum-free media. The selected cells can be grown in serum-free media for at least seven passages to confirm complete stable HEK293 cells that express and secrete full-length, appropriately folded native human proUGN. The gene and mRNA for human preprouroguanylin can be confirmed by PCR, RT-PCR, Southern and Northern analyses. The native human proUGN secreted protein can be confirmed by LC-MS/MS and MRM-MS methods as wells as by Western blot analysis and proUGN ELISA. Purification of proUGN performed by established methods and protein sequencing of the proUGN can be used to confirm the human protein sequence of proUGN. The stably expressed proUGN HEK293 cells do not lose expression of proUGN after twenty passages. Control vector mock-transfected HEK293 cells are performed in parallel and do not express proUGN but serve as control.

Example 2: Development of Monoclonal Antibodies Specific to proUGN.

Hybridoma cells producing antibodies specific for human proUGN can be made by conventional means known to people skilled in the art of cell biology or immunology. Laboratory mice can be immunized with recombinant human protein, peptides conjugated to a carrier protein, plasmids able to express full-length and fragments thereof of the protein, or combinations of these antigen or antigen sources so that the B-cells begin producing antibodies against proUGN. This method can be enhanced by repeat immunization, immunization at different sites such as intradermal, intramuscular, intraperitoneal, and other sites, and through the inclusion of adjuvants such as Freund's adjuvant, saponins, alum, variants of Lipid A, block copolymers, peptide hormones, and many other adjuvants used to promote antigenic responses and antibody development. For example, specific monoclonal antibodies can be created to different overlapping segments of genetically expressed full-length human proUGN - specifically the native form of the protein that is found in the circulation - not a denatured *E. coli* or non-mammalian recombinant protein.

In one embodiment, the present method to develop antibodies specific to proUGN can employ the use of a calibration standard of human proUGN. The calibration standard can be derived from a stably expressed gene in mammalian human kidney cell containing the necessary N-terminal sequence required for protein folding, processing, packaging, and secretion that express and secrete native and appropriately folded and functional proUGN protein (devoid of genetically modified tags that do not occur in nature) without further purification. The calibration standard of proUGN and the immunogen proUGN protein boost(s) or fragments and conjugates of fragments thereof can be used for monoclonal antibody production. In one embodiment, the B-cells from immunized mice can be immortalized by fusing the cells with a suitable stable cell line until the antibody producing hybrid cells are able to reproducibly generate proUGN specific antibodies in high production volume. Antibodies to proUGN do not cross react with the following proteins or fragments thereof or natural occurring or synthetically designed guanylyl cyclase-C agonists or modified analogues or derivatives thereof including but not limited to proguanylin, guanylin, E. coli heat-stable enterotoxins or derivatives thereof, linaclotide, plecanatide, and dolcanatide.

Example 3: Optimized proUGN Assay Methods.

The following method was used for a paired-antibody sandwich ELISA (Figure. 1). Each well (1) of a microtiter/multi-well plate (i.e., 96-well, 384-well) is individually pre-coated with biomarker-specific primary "capture" monoclonal antibodies (mAb) (2). These mAb pre-coated plates are now referred to as Capture Plates.

Specimen from a clinical biological fluid (i.e., serum, plasma, urine, saliva, cerebrospinal fluid, gastrointestinal fluid, amniotic fluid, lung or nasal lavage, and exosomes thereof, etc.) is appropriately diluted 1:10 into Sample Buffer - i.e., 1-part clinical specimen with 9 parts Sample Buffer (Sample Buffer: 10 mM MOPS, protein-free, pH 6.3-7.3). The diluted biological sample is then added to one of the wells of the Capture Plate. The sample is incubated in the well for specified amount of time. In one embodiment, the sample is incubated for approximately one hour (55-65 minutes) in the microtiter well at room temperature (20-25°C). The samples are aspirated out of the well, and the well is washed. In one embodiment, the well is washed and aspirated three times with 350 uL of Plate-Wash Buffer (10-25 mM Tris, 150 mM NaCl, 0.05-0.5% Tween-20, pH 7.8). The analyte, or proUGN (3) or fragments or post-translational modifications thereof, within the biological sample is captured by the capture mAb (3). To the microtiter well (1), the "detection" mAb (4) is added, which is conjugated to a detectable marker. In one embodiment, the detectable or conjugated marker is an enzyme such as horseradish peroxidase (HRP) (5). Note the detection mAb (2) may be conjugated to other enzymes such as alkaline phosphatase or a fluorophore. The detection mAb/enzyme complex is incubated in the microtiter well (1). In one embodiment, the detection complex is incubated in the microtiter well for one hour at room temperature (as described above). Following incubation, the detection mAb solution is aspirated out of the microtiter well, and again, the well is washed with Plate-Wash Buffer. In one embodiment, the well is washed and aspirated three times with 350 uL of Plate-Wash Buffer. In an embodiment using an enzyme as the detectable marker, a solution of enzyme substrate, i.e., TMB (tetramethylbenzidine), is added to the well and incubated. In one embodiment, the incubation is for twenty minutes at room temperature (20-25°C). In this embodiment, the TMB reaction is quenched with the addition of Acid/(0.12N HCL, 0.16M $H_2SO_4$)/STOP-Solution (in the case where TMB is used, the color of the wells will turn from blue to yellow), and the wells / plate is read at an O.D. 450 nm and 650 nm (absorbance readings at A630-A650 act as a reference absorbance only).

Example 4: Expression of proUGN in Serum from Patients with Renal Disease.

Serum samples from patients that were clinically diagnosed as Stage 1, 2, 3, 4, or 5 using the method of classification defined above are tested. Specifically, the CKD sample cohort studies consisted of serum samples from male and female patients formally diagnosed by clinical guidelines.

Estimated GFR was determined according to the following formula described by Levi et al., Ann Intern Med 2009, 150(9), 604-612):

$$eGFR\ (CKD\text{-}EPI) = 141 \times \min(creatinine/k, 1)^{\alpha} \times \max(creatinine/k, 1)^{-1.209} \times 0.993^{Age} \times 1.018\ [\text{if female}]$$

Where *k* is 0.7 for females and 0.9 for males, $\alpha$ is -0.329 for females and -0.411 for males, min indicates the minimum of creatinine/k or 1, and max indicates the maximum of creatinine/k or 1.

The CKD sample cohort was composed of:

14 CKD Stage 1 (mean age 44);
26 CKD Stage 2 (mean age 65);
90 CKD Stage 3 (mean age 68);
31 CKD Stage 4 (mean age 69);
14 CKD Stage 5 (mean age 59);
224 healthy controls (mean age 45).

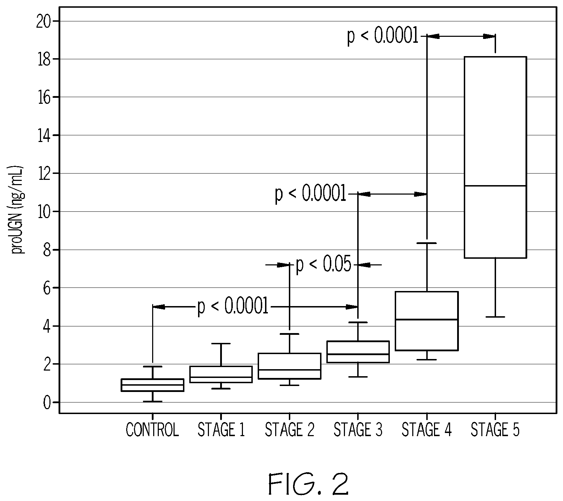

These samples were assayed using the described invention for determining proUGN concentration in biological fluids. The levels of proUGN measured by the assay in renal disease patient's samples were compared to levels of proUGN taken from healthy donors. Significance of results was determined using a Kruskal-Wallis test (J American Stat Assoc, 47, 583-621, 1952). The results demonstrated that the described invention was able to identify renal disease patients in all stages of renal disease (Figure 2). Levels of proUGN compared to the control group were significantly increased in patients clinically diagnosed with "Early CKD" renal disease; Stage 1 ($p<0.001$, n=14), Stage 2 ($p<0.001$, n=26), and Stage 3 ($p<0.0001$, n=90). Additionally, serum from patients with Stage 3 renal disease contained higher levels of proUGN compared to Stage 1 ($p<0.05$, n=14) and Stage 2 ($p<0.005$, n=26), with no significant difference seen between Stage 1 and Stage 2 renal disease patients observed. Furthermore, serum from patients with Stage 4 renal disease contained significantly higher levels of proUGN compared to Stage 3 ($p<0.005$, n=90), and levels from patients with Stage 5 renal disease were significantly increased from Stage 4 ($p<0.0001$, n=31). The results are shown below in Table 1 (SEM, Standard Error of the Mean) and also highlighted in Figure 2.

| Table 1 | proUGN (ng/mL) | | | | | |
|---|---|---|---|---|---|---|
| | Stage 5 | Stage 4 | Stage 3 | Stage 2 | Stage 1 | Controls |
| Average | 11.91 | 459 | 3.13 | 1.91 | 1.90 | 0.91 |
| SEM | 1.39 | 039 | 0.23 | 0.17 | 054 | 0.05 |
| Median | 1131 | 4.31 | 250 | 1.66 | 128 | 0.85 |
| n = | 14 | 31 | 90 | 26 | 14 | 224 |

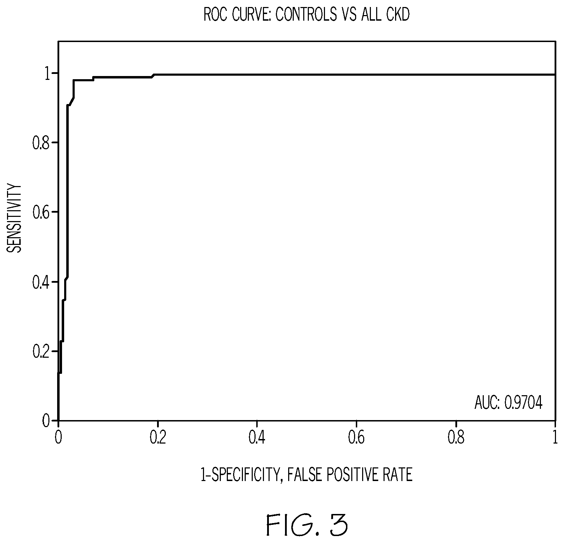

Compared to other biomarkers of kidney disease biomarkers, the invention described herein demonstrated a greater sensitivity for diagnosing renal disease at an early stage. This particular embodiment of the invention demonstrated high detection rates of patient serum samples with clinically diagnosed CKD patients as defined by KDOQI and can diagnose renal disease with 99% sensitivity and 97% specificity (as shown in Example 5) using the predetermined lower threshold value, preferably 1.70 ng/mL, when measured against a normal control group (Figure 3).

The current invention also demonstrates that patient serum samples with CKD Stage 1 / CKD Stage 2 have significantly higher proUGN levels than the normal control group, which is defined by a normal control reference range that is established by the present embodiment of the invention and determined by the formula defined by Stern et al (Essential Medical Statistics, Oxford Blackwell Science, p. 48, 2003); the lower limit of the control reference range of proUGN levels in serum of a healthy patient cohort is calculated to be 0.03 (90% CI, 0.01 - 0.11) ng/mL; wherein the upper limit of the control reference range is calculated to be 1.63 (90% CI, 1.55 - 1.71) ng/mL.

Example 5: Determination of proUGN Threshold Values and Stratification of CKD Stage.

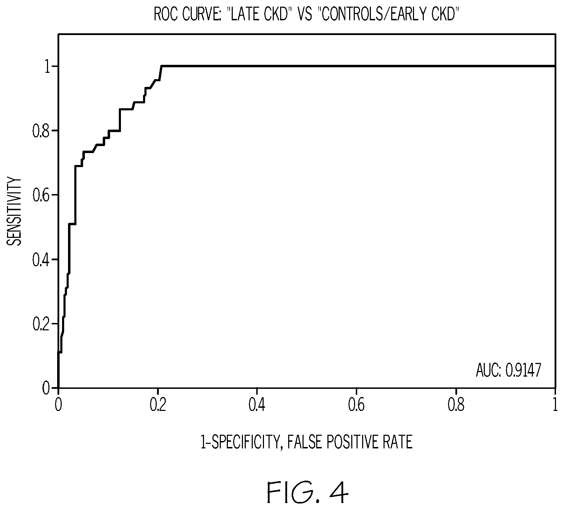

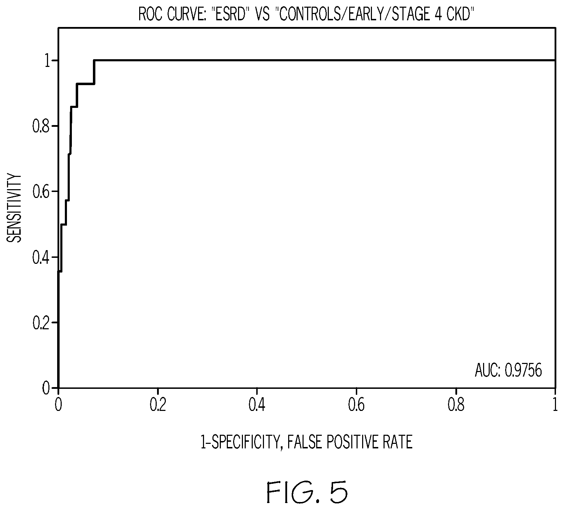

Receiver-operating curve (ROC) analysis was employed to determine the specificity and sensitivity for the proposed invention in the first instance to discriminate between control groups and all CKD patients (Figure 3), in the second instance to discriminate between "Late CKD" and "Controls/Early CKD" (Figure 4), and in the third instance to discriminate between "ESRD" and "Controls/Early CKD/Stage 4 CKD" (Figure 5). In the first instance, area under the curve (AUC) for the ROC for proUGN being employed to discriminate between control groups and all renal disease patients is 0.9704 (i.e., "Lower Threshold" Clinical Cut-off >1.70 ng/mL, Sensitivity = 98.5%, Specificity = 96.9%). In the second instance, AUC for the ROC for proUGN to discriminate between "Late CKD" and "Controls/Early CKD" is 0.9147 (i.e., "Intermediate Threshold" Clinical Cut-off >2.53 ng/mL, Sensitivity = 88.9%, Specificity = 84.7%). In the third instance, AUC for the ROC for proUGN to discriminate between "ESRD" and "Controls/Early CKD/Stage 4 CKD" is 0.9756 (i.e., "Upper Threshold" Clinical Cut-off >5.93 ng/mL, Sensitivity = 92.9%, Specificity = 96.2%). These levels were determined using a cohort of samples collected from a distinct population and therefore control values and reference ranges should be calculated by the end user. The results are shown below in Table 2.

**Table 2**

| ROC Comparison | Predetermined Threshold | Cut-off (ng/mL) | AUC | Sensitivity | Specificity |
|---|---|---|---|---|---|
| Controls vs All CKD | Lower | 1.70 | 0.9704 | 99% | 97% |
| "Late CKD" vs "Control/Early CKD" | Intermediate | 2.53 | 0.9147 | 89% | 85% |
| "ESRD" vs "Control/Early/Stage 4 CKD" | Upper | 5.93 | 0.9756 | 93% | 96% |

The use of quantitative measurements of proUGN in conjunction with other potential markers of CKD, such as but not limited to serum creatinine, allow discrimination between Stage 1 and Stage 2 patients. The main clinical differentiation between Stage 1 and Stage 2 status is GFR or eGFR, calculated from serum creatinine as described above which is correlative by an inverse relationship. Therefore, an elevated proUGN level (above the normal control reference range) combined with elevated creatinine compared to the levels suggested in the instructions for use supplied by the manufacturer that calculate an eGFR within the range of control values 60-89 mL/min/1.73m$^2$ would classify the patient as Stage 2 CKD; wherein an elevated proUGN above the control reference range and below the lower threshold and a serum creatinine concentration within the normal range that calculate an eGFR >90 mL/min/1.73m$^2$ would classify the patient as Stage 1 CKD. Stage 3 patients are classified based on having a proUGN above the lower threshold value and below the intermediate threshold value predicated at a level of 97.5% accuracy, whereas "Late CKD" Stage 4 / 5 are classified based on having a proUGN level above the intermediate threshold value predicted at a level of 85.2% accuracy, whereas "ESRD" is classified based on having a proUGN level above the upper threshold value and is predicted at a level of 96.1% accuracy.

Example 6: Determining Treatment and Management

Once a diagnosis is obtained, the clinician can readily select a treatment regimen, not encompassed by the claimed invention, that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

It is common practice in the medical community to use the transition from CKD Stage 3 to Stage 4 as a demarcation for a change in patient treatment, wherein ESRD indicates the need for dialysis [5, 10]. KDIGO Clinical Practice Guideline for the Evaluation and Management of CKD analyzed management strategies aimed at addressing prevention of CKD progression, decreasing the risk cardiovascular and other diseases, and reduction of mortality rates [5, 55-57].

Clinical Management for "Early CKD" herein defined as Stage 1, 2, or 3. Recommendations include general measures that have been shown to address cardiovascular health and CKD together, and each separately [5, 58]. Addressing CVD risk factors both indirectly and directly impact CKD progression [5]. Strategies include general lifestyle measures, which improve cardiovascular health, BP control, and interruption of the renin-angiotensin-aldosterone system (RAAS). Various treatment modalities, not encompassed by the claimed invention, are separated by targeted complication below for "Early CKD" in general as defined above:

Lifestyle Modifications for "Early CKD"

- Achieving or maintaining a healthy weight (BMI 20 to 25) [59-61].
- Lowering salt intake to <90 mmol (<2 g) per day of sodium (corresponding to 5 g of sodium chloride), unless contraindicated [59].
- Restriction of sodium intake for children with CKD who have hypertension (systolic and/or diastolic blood pressure >95th percentile) or prehypertension (systolic and/or diastolic blood pressure >90th percentile and <95th percentile), following the age-based Recommended Daily Intake [59].
- Supplemental free water and sodium supplements for children with CKD and polyuria to avoid chronic intravascular depletion and to promote optimal growth [59].
- Dietary advice and information in the context of an education program, tailored to severity of CKD and the need to intervene on salt, phosphate, potassium, and protein intake where indicated [5].
- Undertaking an exercise program compatible with cardiovascular health and tolerance, aiming for at least 30 minutes 5 times per week [59].
- Stop smoking [5].
- Limiting alcohol intake to no more than two standard drinks per day for men and no more than one standard drink per day for women [59].
- Avoiding high protein intake (41.3 g/kg/day) in adults with CKD at risk of progression [5].

Glycemic Control for "Early CKD"

- A target hemoglobin A1c (HbA1c) of ~7.0% (53 mmol/mol) +/- 0.5% to prevent or delay progression of the microvascular complications of diabetes, including diabetic kidney disease. However, do not treat <7.0% in patients at risk of hypoglycemia [61].
- In people with CKD and diabetes, glycemic control should be part of a multifactorial intervention strategy addressing blood pressure control and cardiovascular risk, promoting the use of angiotensin- converting enzyme inhibition or angiotensin receptor blockade, statins, and antiplatelet therapy where clinically indicated [61].

Blood Pressure Management (without Diabetes Mellitus) for "Early CKD"

- Individualize BP targets and agents according to age, co-existent cardiovascular disease and other comorbidities, risk of progression of CKD, and tolerance of treatment. Inquire about postural dizziness and check for postural hypotension regularly when treating CKD patients with BP-lowering drugs [59].
- Non-diabetic adults with urine albumin excretion <30 mg per 24 hours (or equivalent*) whose office BP is consistently >140 mm Hg systolic or >90 mm Hg diastolic be treated with BP-lowering drugs to maintain a BP that is consistently ≤140 mm Hg systolic and ≤90 mm Hg diastolic [59].
- Non-diabetic adults and urine albumin excretion of 30 to 300 mg per 24 hours (or equivalent*) whose office BP is consistently >130 mm Hg systolic or >80 mm Hg diastolic be treated with BP-lowering drugs to maintain a BP that is consistently ≤130 mm Hg systolic and ≤80 mm Hg diastolic [59].
- Non-diabetic adults with urine albumin excretion >300 mg per 24 hours (or equivalent*) whose office BP is consistently >130 mm Hg systolic or >80 mm Hg diastolic be treated with BP-lowering drugs to maintain a BP that is consistently ≤130 mm Hg systolic and ≤80 mm Hg diastolic [59].
- That an angiotensin II receptor blocker (ARB) or angiotensin converting enzyme inhibitor (ACE-I) be used in non-diabetic adults and urine albumin excretion >300 mg per 24 hours (or equivalent*) in whom treatment with BP-lowering drugs is indicated [59].

  *Approximate equivalents for urinary albumin excretion rate per 24 hours are as follows: expressed as protein excretion rate per 24 hours, urine albumin/creatinine ratio (UACR), protein/ creatinine ratio, and protein reagent strip results.

Blood Pressure Management (with Diabetes Mellitus) for "Early CKD"

- Individualize BP targets and agents according to age, co-existent cardiovascular disease and other comorbidities, risk of progression of CKD, presence or absence of retinopathy (in CKD patients with diabetes) and tolerance of treatment. Inquire about postural dizziness and check for postural hypotension regularly when treating CKD patients with BP-lowering drugs [59, 61].
- That adults with diabetes and with urine albumin excretion <30 mg per 24 hours (or equivalent*) whose office BP is consistently >140 mm Hg systolic or >90 mm Hg diastolic be treated with BP-lowering drugs to maintain a BP that is consistently ≤140 mm Hg systolic and ≤90 mm Hg diastolic [59].
- That adults with diabetes and urine albumin excretion >30 mg per 24 hours (or equivalent*) whose office BP is consistently >130 mm Hg systolic or >80 mm Hg diastolic be treated with BP-lowering drugs to maintain a BP that is consistently ≤130 mm Hg systolic and ≤80 mm Hg diastolic [59].
- That an ARB or ACE-I be used in adults with diabetes and urine albumin excretion >30 mg per 24 hours (or equivalent*) [59, 61]. *Approximate equivalents for albumin excretion rate per 24 hours are as follows: expressed as protein excretion rate per 24 hours, urine albumin/creatinine ratio (UACR), protein/ creatinine ratio, and protein reagent strip results.

Cardiovascular Disease for "Early CKD"

- Adults with CKD at risk for atherosclerotic events be offered treatment with antiplatelet agents unless there is an increased bleeding risk that needs to be balanced against the possible cardiovascular benefits [5].
- In people with GFR <60 ml/min/1.73 m$^2$ (GFR categories G3a-G5), we recommend that serum concentrations of brain natriuretic peptide (BNP) or NT-proBNP be interpreted with caution and in relation to GFR with respect to diagnosis of heart failure and assessment of volume status [5].
- In people with GFR <60 ml/min/1.73 m$^2$ (GFR categories G3a-G5), we recommend that serum concentrations of troponin be interpreted with caution and in relation to GFR with respect to diagnosis of acute coronary syndrome [5].
- In adults aged ≥50 years with CKD and eGFR ≥60 ml/min/1.73 m$^2$ (i.e. Stage 1 or 2, GFR categories G1-G2) should be treated with a statin [62].

Peripheral Artery Disease for "Early CKD"

- Adults with CKD be regularly examined for signs of peripheral arterial disease and be considered for usual approaches to therapy [5].
- Adults with CKD and diabetes should undergo regular podiatric assessment [5].

Medication Management, Imaging, and Patient Safety for "Early CKD"

- Where precision is required for dosing (due to narrow therapeutic or toxic range) and/or estimates may be unreliable (e.g., due to low muscle mass), methods based upon cystatin C or direct measurement of GFR should be used [5, 63].
- Temporary discontinuation of potentially nephrotoxic and renally-excreted drugs in people with a GFR <60 ml/min/1.73 m$^2$ (GFR categories G3a-G5) who have serious intercurrent or concurrent illness that increases the risk of AKI. These agents include but are not limited to: RAAS blockers (including ACE-I's, ARBs, aldosterone inhibitors, direct renin inhibitors), diuretics, NSAIDs, metformin, lithium, and digoxin [5, 63].
- Metformin should be continued in people with GFR ≥45 ml/min/1.73 m$^2$ (GFR categories G1-G3a) [5].
- Oral phosphate-containing bowel preparations in people with a GFR <60 ml/min/1.73 m$^2$ (GFR categories G3a-G5) should not be used [5].

Infections and Vaccinations for "Early CKD"

- All adults with CKD are offered annual vaccination with influenza vaccine, unless contraindicated [5].
- All adults with CKD who have received pneumococcal vaccination are offered revaccination within 5 years [5].

Clinical management, not encompassed by the claimed invention, for "Late CKD" herein defined as Stages 4 and 5. Treatment modalities, guidelines and recommendations for "Late CKD" Stages, in general, as defined above, are separated by targeted comorbid complication below:
Lifestyle Modifications for "Late CKD"

- Protein intake should be lowered to 0.8 g/kg/day in adults with diabetes or without diabetes and GFR <30 ml/min/1.73 m$^2$ with appropriate patient education [5].

Cardiovascular Disease for "Late CKD"

- In adults aged ≥50 years with a baseline eGFR <60 ml/min/1.73 m$^2$ (GFR categories G3a-G5 but not treated with chronic dialysis or kidney transplantation), it is recommended to treat with a statin or statin/ezetimibe combination therapy [62, 64].

- In adults aged 18-49 years with CKD but not treated with chronic dialysis or kidney transplantation, statin treatment should be initiated in the presence of one or more of the following conditions: known coronary disease (myocardial infarction or coronary revascularization), diabetes mellitus, prior ischemic stroke, or estimated 10-year incidence of coronary death or non-fatal myocardial infarction >10% [62].

Anemia for "Late CKD"

- Diagnose anemia in adults and children >15 years with CKD when the hemoglobin (Hb) concentration is <13.0 g/dl (<130 g/l) in males and <12.0 g/dl (<120 g/l) in females [5, 14, 65].
- Diagnose anemia in children with CKD if Hb concentration is <11.0 g/dl (<110 g/l) in children 0.5-5 years, <11.5 g/dl (115 g/l) in children 5-12 years, and <12.0 g/dl (120 g/l) in children 12-15 years [5].
- Iron and recombinant erythropoietin and its synthetic derivatives (epoetin alfa, epoetin beta, darbepoetin alfa, methoxy polyethylene glycolepoetin beta; collectively known as erythropoiesis-stimulating agents) have been shown to reduce the need for blood transfusion in people with CKD, particularly when used in combination [65].

Mineral Bone Disease for "Late CKD"

- In people with GFR <45 ml/min/1.73 m$^2$ (GFR categories G3b-G5, previously referred to <2012 as Stages 3b-5), serum phosphate concentrations should be maintained in the normal range according to local laboratory reference values [5, 65].
- In people with GFR <45 ml/min/1.73 m$^2$ (GFR categories G3b-G5) and PTH above the upper normal limit of the assay are first evaluated for hyperphosphatemia, hypocalcemia, and vitamin D deficiency [5].
- Oral activated vitamin D treatments or vitamin D analogs be prescribed in the presence of documented deficiency to suppress elevated PTH concentrations in people [65].
- Dietary restriction of phosphate and the use of either calcium or non-calcium-based phosphate binders to obtain serum phosphate concentrations of between 0.87 mmol/L and 1.49 mmol/L [65].
- Prescribe bisphosphonate treatment in people with GFR <30 ml/min/1.73 m$^2$ (GFR categories G4-G5) only in the presence of strong clinical rationale [5].

Acidosis for "Late CKD"

- Those with CKD and serum bicarbonate concentrations <22 mmol/l should be treated with oral bicarbonate supplementation to maintain serum bicarbonate within the normal range, unless contraindicated. [5, 65].

Medication Management, Imaging, and Patient Safety for "Late CKD"

- Metformin use should be reviewed in those with GFR 30-44 ml/min/1.73 m$^2$ and it should be discontinued in people with GFR <30 ml/min/1.73 m$^2$ (GFR categories G4-G5) [5].
- People with a GFR <30 ml/min/1.73 m$^2$ (GFR categories G4-G5) who require gadolinium-containing contrast media are preferentially offered a macrocyclic chelate preparation [5].
- Gadolinium-containing contrast media in people with GFR <15 ml/min/1.73 m$^2$ (GFR category G5) should not be used unless there is no alternative appropriate test [5].

Infections and Vaccination for "Late CKD"

- All adults with eGFR <30 ml/min/1.73 m$^2$ (GFR categories G4-G5) and those at high risk of pneumococcal infection (e.g., nephrotic syndrome, diabetes, or those receiving immunosuppression) receive vaccination with polyvalent pneumococcal vaccine unless contraindicated [5].

- All adults with high risk of progression CKD or have GFR <30 ml/min/1.73 m$^2$ (GFR categories G4-G5) be immunized against hepatitis B and the response confirmed by appropriate serological testing [5].

**REFERENCES CITED.**

**U.S Patent Documents**

| Patent Number | Issue Date | Inventor |
|---|---|---|
| 8173596 | May 2012 | Goy et al. |
| 5234933 | August 1993 | Marnett et al. |
| 5326902 | July 1994 | Seipp et al. |
| 5489670 | February 1996 | Currie et al. |
| 5879656 | March 1999 | Waldman |
| 6180082 | January 2001 | Woltering et al. |
| 7522762 | April 2009 | Rea et al. |
| 2009/0181852 A1 | July 2009 | Clark et al |
| 2011/0306125 A1 | December 2011 | Kessler |
| 8129191 | March 2012 | Sheard et al. |
| 8361389 | January 2013 | Sheard et al. |
| 9090698 | July 2015 | Mukherj ee et al. |
| 9708371 | July 2017 | Kessler et al. |
| 9814752 | November 2017 | Shailubhai et al. |
| 9845362 | December 2017 | Mukherj ee et al. |
| 10118946 | November 2018 | Shailubhai et al. |
| 10232011 | March 2019 | Comiskey et al. |

| Application | Number Filing Date | Patent Number | Issue Date |
|---|---|---|---|
| 12/143,769 | June 21, 2008 | 7,977,110 | July 12, 2011 |
| 13/118,932 | May 31, 2011 | 8,602,996 | December 10,2013 |
| 13/945,477 | July 18, 2013 | 8,815,602 | August 26, 2014 |
| 13/128,392 | November 10, 2009 | 8,993,250 | March 31, 2015 |
| 13/061,446 | August 28, 2009 | 9,057,735 | June 16, 2015 |
| 13/061,446 | August 28, 2009 | 9,057,735 | June 16, 2015 |
| 13/125,631 | October 22, 2009 | 9,128,107 | September 8, 2015 |
| 14/041,682 | September 30, 2013 | 9,232,897 | January 12, 2016 |
| 14/738,904 | June 14, 2015 | 9,417,250 | August 16, 2016 |
| 13/793,833 | March 11, 2013 | 9,689,826 | June 27, 2017 |
| 15/664,476 | July 31, 2017 | 10,156,564 | December 18,2018 |
| 15/478,007 | April 3, 2017 | 10,269,447 | April 23, 2019 |
| 15/034,338 | October 31, 2014 | 10,274,502 | April 30, 2019 |
| 14/421,382 | August 12, 2013 | 10,444,248 | October 15, 2019 |

**Foreign Patent Documents**

| WO 93/25521 | Dec 1993 | WO |
|---|---|---|
| WO 97/06258 | Feb 1997 | WO |

(continued)

| WO 97/20049 | Jun 1997 | WO |
|---|---|---|

**Other References**

1. NIH, N.I.o.H., 2018 USRDS Annual Data Report: Epidemiology of Kidney Disease in the United States., N. National Institutes of Health, Editor. 2018: Bethesda, MD.

2. U.S. Renal Data System (USRDS), USRDS, 2014 annual data report: An overview of the epidemiology of kidney disease in the United States, N. NIH, Editor. 2014: Bethesda, MD.

3. National Kidney Foundation, KDOQI Clinical Practice Guideline for Diabetes and CKD: 2012 Update. Am J Kidney Dis, 2012. 60(5): p. 850-86.

4. Satchidanand, N., et al., Positive Predictive Value of a Single Assessment of Estimated GFR in the Diagnosis of Chronic Kidney Disease. South Med J, 2016. 109(6): p. 351-5.

5. KDIGO CKD Work Group 2012, KDIGO 2012 clinical practice guideline for the evaluation and management of chronic kidney disease. Kidney International Suppl, 2013. 3(1): p. 1-150.

6. McTaggart, M.P., et al., The diagnostic accuracy of a urine albumin-creatinine ratio point-of-care test for detection of albuminuria in primary care. Am J Kidney Dis, 2012. 60(5): p. 787-94.

7. van Stralen, K.J., et al., Diagnostic methods I: sensitivity, specificity, and other measures of accuracy. Kidney Int, 2009. 75(12): p. 1257-1263.

8. Waikar, S.S., et al., Biological Variability of Estimated GFR and Albuminuria in CKD. Am J Kidney Dis, 2018. 72(4): p. 538-546.

9. Gaitonde, D.Y., D.L. Cook, and I.M. Rivera, Chronic Kidney Disease: Detection and Evaluation. Am Fam Physician, 2017. 96(12): p. 776-783.

10. Fox, C.H., et al., A quick guide to evidence-based chronic kidney disease care for the primary care physician. Postgraduate Medicine, 2008. 120(2): p. 1-6.

11. Ravizza, S., et al., Predicting the early risk of chronic kidney disease in patients with diabetes using real-world data. Nat Med, 2019. 25(1): p. 57-59.

12. Naresh, C.N., et al., Day-to-day variability in spot urine albumin-creatinine ratio. Am J Kidney Dis, 2013. 62(6): p. 1095-101.

13. Ryan, T.P., et al., Chronic kidney disease prevalence and rate of diagnosis. Am J Med, 2007. 120(11): p. 981-6.

14. Inker, L.A., et al., KDOQI US commentary on the 2012 KDIGO clinical practice guideline for the evaluation and management of CKD. Am J Kidney Dis, 2014. 63(5): p. 713-35.

15. Coresh, J., et al., Current CKD Definition Takes into Account Both Relative and Absolute Risk. J Am Soc Nephrol, 2019.

16. Prevention, C.f.D.C.a. Chronic Kidney Disease in the United States 2019. Centers for Disease Control and Prevention, US Department of Health and Human Services, 2019. 1, 1.

17. Okusa, M.D., A hidden epidemic: More than 850 million suffer from kidney diseases worldwide, organizations report. Kidney News, 2019. 5(5): p. 18-19.

18. Levey, A.S., et al., National Kidney Foundation practice guidelines for chronic kidney disease: Evaluation, classification, and stratification. Ann Intern Med, 2003. 139(2): p. 137-47.

19. National Kidney Foundation. National Kidney Foundation: Kidney Disease. National Kidney Foundation: Kidney disease [Website] 2008 [cited 2008 11/15/2008]; Available from: http://www.kidney.org/kidneydisease/.

20. Narva, A.S., The National Kidney Disease Education Program and other related efforts in the United States. Scand J Clin Lab Invest Suppl, 2008. 241: p. 12-5.

21. CDC. National Health and Examination Survey. National Center for Health Statistics, NHANES 2012 [cited 2020 Jan. 10]; Available from: www.cdc.gov/nchs/nhanes.

22. NIH, U.S.D.o.H.a.H.S. Healthy People 2020. Healthy People.Gov 2011 July 21, 2011 [cited 2011 July 21, 2011]; Healthy People provides science-based, 10-year national objectives for improving the health of all Americans.]. Available from: http://healthypeople.gov/2020/about/default.aspx.

23. NIH, K.N. Healthy People 2010 Objectives: Chronic Kidney Disease. HealthPeople.Gov 2010 Feburary 24, 2011 [cited 2011 July 21, 2011]; Reduce the incidence, morbidity, mortality and health care costs of chronic kidney disease.]. Available from: http://www2.niddk.nih.gov/AboutNIDDK/Organization/Divisions/KUH/KUHKidney HP2010.htm.

24. Pergola, P.E., et al., Effect of bardoxolone methyl on kidney function in patients with T2D and Stage 3b-4 CKD. Am J Nephrol, 2011. 33(5): p. 469-76.

25. Pergola, P.E., et al., Bardoxolone Methyl and Kidney Function in CKD with Type 2 Diabetes. N Engl J Med, 2011.

365(4): p. 327-336.

26. Forte, L.R., Jr., Uroguanylin: Physiological role as a natriuretic hormone. J Am Soc Nephrol, 2005. 16(2): p. 291-2.

27. Forte, L.R., X. Fan, and F.K. Hamra, Salt and water homeostasis: Uroguanylin is a circulating peptide hormone with natriuretic activity. Am J Kidney Dis, 1996. 28(2): p. 296-304.

28. Forte, L.R., et al., Guanylin peptides: renal actions mediated by cyclic GMP. Am J Physiol Renal Physiol, 2000. 278(2): p. F180-91.

29. Forte, L.R., Jr., Uroguanylin and guanylin peptides: pharmacology and experimental therapeutics. Pharmacol Ther, 2004. 104(2): p. 137-62.

30. Kinoshita, H., et al., Urine and plasma levels of uroguanylin and its molecular forms in renal diseases. Kidney International, 1997. 52(4): p. 1028-1034.

31. Hess, R., et al., GCAP-II: isolation and characterization of the circulating form of human uroguanylin. FEBS Lett, 1995. 374(1): p. 34-8.

32. Klodt, J., et al., Synthesis, biological activity and isomerism of guanylate cyclase C-activating peptides guanylin and uroguanylin. J Pept Res, 1997. 50(3): p. 222-30.

33. Li, Z., et al., Purification, cDNA sequence, and tissue distribution of rat uroguanylin. Regul Pept, 1997. 68(1): p. 45-56.

34. Perkins, A., M.F. Goy, and Z. Li, Uroguanylin is expressed by enterochromaffin cells in the rat gastrointestinal tract. Gastroenterology, 1997. 113(3): p. 1007-14.

35. Nakazato, M., et al., Tissue distribution, cellular source, and structural analysis of rat immunoreactive uroguanylin. Endocrinology, 1998. 139(12): p. 5247-54.

36. Nakazato, M., Guanylin family: new intestinal peptides regulating electrolyte and water homeostasis. J Gastroenterol, 2001. 36(4): p. 219-25.

37. Sindic, A., Current understanding of guanylin peptides actions. ISRN Nephrol, 2013. 2013: p. 813648.

38. Sindic, A., et al., Guanylin and uroguanylin regulate electrolyte transport in isolated human cortical collecting ducts. Kidney Int, 2005. 67(4): p. 1420-7.

39. Carrithers, S.L., et al., Site-specific effects of dietary salt intake on guanylin and uroguanylin mRNA expression in rat intestine. Regul Pept, 2002. 107(1-3): p. 87-95.

40. Carrithers, S.L., et al., Guanylyl cyclase-C (GC-C) receptor mRNA distribution in the rat nephron. Journal of Investigative Medicine, 2000. 48(2): p. 20.

41. Miyazato, M., et al., Uroguanylin gene expression in the alimentary tract and extra-gastrointestinal tissues. FEBS Letters, 1996. 398(2-3): p. 170-174.

42. Kulaksiz, H. and Y. Cetin, Uroguanylin and guanylate cyclase C in the human pancreas: expression and mutuality of ligand/receptor localization as indicators of intercellular paracrine signaling pathways. Journal of Endocrinology, 2001. 170(1): p. 267-275.

43. Mueller, T. and B. Dieplinger, The guanylin peptide family and the proposed gastrointestinal-renal natriuretic signaling axis. Kidney Int, 2012. 82(12): p. 1253-5.

44. Valentino, M.A., et al., A uroguanylin-GUCY2C endocrine axis regulates feeding in mice. J Clin Invest, 2011. 121(9): p. 3578-88.

45. Moss, N.G., et al., Uroguanylin, an intestinal natriuretic peptide, is delivered to the kidney as an unprocessed propeptide. Endocrinology, 2008. 149(9): p. 4486-98.

46. Qian, X., et al., Circulating prouroguanylin is processed to its active natriuretic form exclusively within the renal tubules. Endocrinology, 2008. 149(9): p. 4499-509.

47. Qian, X., et al., The rat kidney contains high levels of prouroguanylin (the uroguanylin precursor) but does not express GC-C (the enteric uroguanylin receptor). Am J Physiol Renal Physiol, 2011. 300(2): p. F561-73.

48. Carrithers, S.L., et al., Natriuretic and kaliuretic properties of guanylin and uroguanylin in vivo. Journal of Investigative Medicine, 1999. 47(4): p. 183A-183A.

49. Carrithers, S.L., et al., Renal effects of uroguanylin and guanylin in vivo. Brazilian Journal of Medical and Biological Research, 1999. 32(11): p. 1337-1344.

50. Forte, L.R., et al., Mechanisms of guanylin action via cyclic GMP in the kidney. Annual Review of Physiology, 2000. 62: p. 673-695.

51. Carrithers, S.L., et al., Guanylin and uroguanylin induce natriuresis in mice lacking guanylyl cyclase-C receptor. Kidney Int, 2004. 65(1): p. 40-53.

52. Carrithers, S.L., et al., Guanylyl cyclase-C receptor mRNA distribution along the rat nephron. Regul Pept, 2000. 95(1-3): p. 65-74.

53. Levey, A.S., et al., Definition and classification of chronic kidney disease: a position statement from Kidney Disease: Improving Global Outcomes (KDIGO). Kidney Int, 2005. 67(6): p. 2089-100.

54. Murphy, D., et al., Trends in prevalence of chronic kidney disease in the United States. Ann Intern Med, 2016. 165(7): p. 473-481.

55. Hui, X., et al., CKD and cardiovascular disease in the Atherosclerosis Risk in Communities (ARIC) study: interactions with age, sex, and race. Am J Kidney Dis, 2013. 62(4): p. 691-702.

56. Yang, M., et al., Complications of progression of CKD. Adv Chronic Kidney Dis, 2011. 18(6): p. 400-405.

57. Andrassy, K.M., Commentary of 'KDIGO 2012 clinical practice guideline for the evaluation and management of chronic kidney disease.'. Kidney Int'1, 2013. 84(3): p. 622-623.

58. Qaseem, A., et al., Screening, monitoring, and treatment of stage 1 to 3 chronic kidney disease: A clinical practice guideline from the American College of Physicians. Ann Intern Med, 2013. 159(12): p. 835-47.

59. Taler, S.J., et al., KDOQI US commentary on the 2012 KDIGO clinical practice guideline for management of blood pressure in CKD. Am J Kidney Dis, 2013. 62(2): p. 201-13.

60. KDOQI, Clinical practice guidelines for chronic kidney disease: Evaluation, classification, and stratification. Am J Kidney Dis, 2002. 39: p. S1-S266.

61. KDOQI CKD Work Group Update, KDOQI Clinical practice Guideline for Diabetes and CKD: 2012 Update. Am J Kidney Dis, 2012. 60(5): p. 850-886.

62. KDIGO Lipid Work Group, KDIGO clinical practice guideline for lipid management in chronic kidney disease. Kidney Int'l Suppl, 2013. 3(3): p. 159-305.

63. Palevsky, P.M., et al., KDOQI US commentary on the 2012 KDIGO clinical practice guideline for acute kidney injury. Am J Kidney Dis, 2013. 61(5): p. 649-72.

64. Mefford, M.T., et al., Trends in Statin Use Among US Adults With Chronic Kidney Disease, 1999-2014. J Am Heart Assoc, 2019. 8(2): p. e010640.

65. KDIGO CKD Work Group 2017, KDIGO 2017: Clinical practice guideline update for the diagnosis, evaluation, prevention, and treatment of chronic kidney disease - Mineral and Bone Disorder (CKD-MBD). Kidneys, 2017. 6(3): p. 149-154.

**Claims**

1. A method of

   (i) diagnosing a renal dysfunction or disease and/or renal complications of a disease in a subject, or
   (ii) diagnosing or monitoring kidney function in a subject, or
   (iii) diagnosing kidney disease or renal complications prior to the onset of kidney degeneration in a subject, or
   (iv) predicting or monitoring the risk of an adverse event in a subject with kidney disease wherein said adverse event is selected from the group comprising worsening of kidney dysfunction including kidney failure, loss of kidney function, development of related comorbid condition, end-stage kidney disease, or death, or
   (v) predicting or monitoring the success of a therapy or intervention in a subject with kidney disease, the method comprising:

   (a) measuring a level or amount of prouroguanylin (proUGN) in a biological sample,
   (b) comparing the measurement to a control reference range or threshold value,
   (c) determining whether the measurement of proUGN is above the control reference range or threshold value.

2. The method of claim 1, wherein the control reference range or threshold value is a normal range of values for subjects without renal disease.

3. The method of claim 1 or 2, wherein measuring the level or amount of proUGN in the biological sample comprises determining the level of prouroguanylin (proUGN), or fragments or post-translational modifications thereof.

4. The method of claim 3, wherein measuring the level or amount of proUGN in the biological sample comprises using at least one binding moiety specific for prouroguanylin (proUGN), or fragments or post-translational modifications thereof.

5. The method of claim 4, wherein the at least one binding moiety is an antibody, an antibody fragment, or a non-Ig-scaffold binding protein.

6. The method of claim 5, wherein the at least one binding moiety binds to a region of at least 4 amino acids of full-length prouroguanylin (proUGN), an N-terminal fragment of proUGN, or a C-terminal fragment of proUGN.

7. The method of claim 6, wherein the full-length prouroguanylin (proUGN) has the sequence **vyiqyqgfrvqlesmkkl sdleaqwapsprlqaqsllpavchhpalpqdlqpvcasqeassifktlrtiand dcelcvnvactgcl** (SEQ ID NO: 1), the N-terminal fragment of proUGN has the sequence **vyiqyqgfrvqlesmkklsdleaqwapsprlqaqsllpavchhpalpqdlqpvcasqeass ifktlrtia** (SEQ ID NO: 2), and the C-terminal fragment of proUGN has the sequence **tianddcelcvnvactgcl** (SEQ ID NO: 3) and the C-terminal fragment of proUGN has the sequence **nddcelcvnvactgcl** (SEQ ID NO: 4).

8. The method of any one of the preceding claims, wherein step (a) is a quantitative measure of prouroguanylin (proUGN) that comprises an immunological assay selected from the group consisting of enzyme linked immunosorbent assay (ELISA), lateral flow assay, counting immunoassay (CIA), chemiluminescence immunoassay (CLIA), radioimmunoassay (RIA), immunoradiometric assay (IRMA) and Western blot, preferably ELISA.

9. The method of any one of the preceding claims, wherein the biological sample is selected from the group consisting of urine, blood, serum, plasma, saliva, cerebrospinal fluid, amniotic fluid, lung or nasal lavage, and gastrointestinal fluid.

10. The method of claim 8, wherein the ELISA comprises:

transferring the biological sample to a capture plate comprising an immobilized proUGN monoclonal antibody; removing the biological sample from the capture plate; adding a second proUGN monoclonal antibody to the capture plate, the second proUGN monoclonal antibody conjugated to a detectable marker; and determining a level of the detectable marker.

11. The method of any one of the preceding claims, wherein the control reference range or threshold value corresponds to 0.91 ng/mL proUGN.

12. The method of claim 10, wherein the level of the detectable marker above the control reference range or threshold value is indicative of stage 1, stage 2, stage 3, stage 4, or stage 5 chronic kidney disease.

13. The method of claim 8, wherein the lateral flow assay comprises capturing the proUGN with a capture monoclonal antibody, and binding a detection monoclonal antibody to the proUGN.

14. The method of any one of the preceding claims, wherein the disease is chronic kidney disease (CKD).

15. A kit for

(i) diagnosing a renal dysfunction or disease and/or renal complications of a disease in a subject, or
(ii) diagnosing or monitoring kidney function in a subject, or
(iii) diagnosing kidney disease or renal complications prior to the onset of kidney degeneration in a subject, or
(iv) predicting or monitoring the risk of an adverse event in a subject with kidney disease wherein said adverse event is selected from the group comprising worsening of kidney dysfunction including kidney failure, loss of kidney function, development of related comorbid condition, end-stage kidney disease, or death, or
(v) predicting or monitoring the success of a therapy or intervention in a subject with kidney disease,

encompassing one or more containers, comprising:

(a) a substrate having a surface and a first proUGN antibody adhered to the surface;
(b) a second proUGN antibody comprising a detectable marker;
wherein at least one of the first and second proUGN antibodies are monoclonal antibodies which specifically binds to human proUGN, or a fragment or post-translational modification thereof, wherein the human proUGN, or the fragment or post-translational modification thereof is secreted from a human kidney cell; and
(c) instructions for performing one or more of the diagnostic and/or prognostic correlations recited in any one of (i) to (v).

16. The kit of claim 15 wherein the first and second proUGN antibodies are monoclonal antibodies.

17. The kit of claim 15 or 16, wherein the first and second proUGN antibodies are capable of binding proUGN having the sequence as set forth in SEQ ID NO:1.

**18.** The kit of any one of claims 15 to 17, wherein the disease is chronic kidney disease (CKD).

**Patentansprüche**

**1.** Verfahren zur

(i) Diagnose einer Nierenfunktionsstörung oder -krankheit und/oder von Nierenkomplikationen einer Krankheit bei einem Subjekt, oder
(ii) Diagnose oder Überwachung der Nierenfunktion bei einem Subjekt, oder
(iii) Diagnose einer Nierenkrankheit oder von Nierenkomplikationen vor dem Einsetzen der Nierendegeneration bei einem Subjekt, oder
(iv) Vorhersage oder Überwachung des Risikos eines unerwünschten Ereignisses bei einem Subjekt mit Nierenkrankheit, wobei das unerwünschte Ereignis ausgewählt ist aus der Gruppe, umfassend eine Verschlechterung von Nierenfunktionsstörung, einschließlich Nierenversagen, Verlust der Nierenfunktion, Entwicklung eines verwandten komorbiden Zustands, Nierenkrankheit im Endstadium oder Tod, oder
(v) Vorhersage oder Überwachung des Erfolgs einer Therapie oder Intervention bei einem Subjekt mit Nierenkrankheit, das Verfahren umfassend:

(a) Messen eines Pegels oder einer Menge von Prouroguanylin (proUGN) in einer biologischen Probe,
(b) Vergleichen des Messwerts mit einem Referenzbereich oder Schwellenwert,
(c) Bestimmen, ob der Messwert von proUGN über dem Referenzbereich oder Schwellenwert ist.

**2.** Das Verfahren nach Anspruch 1, wobei der Referenzbereich oder der Schwellenwert ein normaler Bereich von Werten für Subjekte ohne Nierenkrankheit ist.

**3.** Das Verfahren nach Anspruch 1 oder 2, wobei ein Messen des Pegels oder der Menge von proUGN in der biologischen Probe ein Bestimmen des Pegels von Prouroguanylin (proUGN) oder Fragmenten oder posttranslationalen Modifikationen davon umfasst.

**4.** Das Verfahren nach Anspruch 3, wobei ein Messen des Pegels oder der Menge von proUGN in der biologischen Probe ein Verwenden mindestens einer für Prouroguanylin (proUGN) spezifischen Bindungsgruppierung oder von Fragmenten oder posttranslationalen Modifikationen davon umfasst.

**5.** Das Verfahren nach Anspruch 4, wobei die mindestens eine Bindungsgruppierung ein Antikörper, ein Antikörperfragment oder ein nicht-Ig-Gerüst-Bindungsprotein ist.

**6.** Das Verfahren nach Anspruch 5, wobei die mindestens eine Bindungsgruppierung an eine Region von mindestens 4 Aminosäuren von Prouroguanylin (proUGN) voller Länge, ein N-terminales Fragment von proUGN oder ein C-terminales Fragment von proUGN bindet.

**7.** Das Verfahren nach Anspruch 6, wobei das Prouroguanylin (proUGN) voller Länge die Sequenz **vyiqyqgfrvqles mkklsdleaqwapsprlqaqsllpavchhpalpqdlqpvcasqeassifktlrtian ddcelcvnvactgcl** (SEQ ID NO: 1) aufweist, das N-terminale Fragment von proUGN die Sequenz **vyiqyqgfrvqlesmkklsdleaqwapsprlqaqsllpavchhpalpqdlqpv casqeassifktlrtia** (SEQ ID NO: 2) aufweist, und das C-terminale Fragment von proUGN die Sequenz **tianddcelcvnvactgcl** (SEQ ID NO: 3) aufweist und das C-terminale Fragment von proUGN die Sequenz **nddcelcvnvactgcl** (SEQ ID NO: 4) aufweist.

**8.** Das Verfahren nach einem der vorherigen Ansprüche, wobei Schritt (a) eine quantitative Messung von Prouroguanylin (proUGN) ist, die einen immunologischen Assay umfasst, der ausgewählt ist aus der Gruppe, bestehend aus Enzymimmunoassay (ELISA), Lateral-Flow-Assay, Counting-Immunoassay (CIA), Chemilumineszenz-Immunoassay (CLIA), Radioimmunoassay (RIA), immunoradiometrischen Assay (IRMA) und Western Blot, vorzugsweise ELISA

**9.** Das Verfahren nach einem der vorherigen Ansprüche, wobei die biologische Probe ausgewählt ist aus der Gruppe, bestehend aus Urin, Blut, Serum, Plasma, Speichel, Liquor, Zerebrospinalflüssigkeit, Lungen- oder Nasenspülung und Magen-Darm-Flüssigkeit.

10. Das Verfahren nach Anspruch 8, wobei der ELISA Folgendes umfasst:

Überführen der biologischen Probe auf eine Fangplatte, umfassend einen immobilisierten monoklonalen proUGN-Antikörper;
Entfernen der biologischen Probe von der Fangplatte;
Hinzufügen eines zweiten monoklonalen proUGN-Antikörpers zu der Fangplatte, wobei der zweite monoklonale proUGN-Antikörper mit einem detektierbaren Marker konjugiert ist; und
Bestimmen eines Pegels des detektierbaren Markers.

11. Das Verfahren nach einem der vorherigen Ansprüche, wobei der Referenzbereich oder Schwellenwert 0,91 ng/ml proUGN entspricht.

12. Das Verfahren nach Anspruch 10, wobei der Pegel des detektierbaren Markers oberhalb des Referenzbereichs oder Schwellenwerts indikativ für eine chronische Nierenkrankheit in Stadium 1, Stadium 2, Stadium 3, Stadium 4 oder Stadium 5 ist.

13. Das Verfahren nach Anspruch 8, wobei der Lateral-Flow-Assay ein Fangen des proUGN mit einem monoklonalen Fang-Antikörper und ein Binden eines monoklonalen Detektions-Antikörpers an das proUGN umfasst.

14. Das Verfahren nach einem der vorherigen Ansprüche, wobei die Krankheit eine chronische Nierenkrankheit (CKD) ist.

15. Kit zur

(i) Diagnose einer Nierenfunktionsstörung oder -krankheit und/oder von Nierenkomplikationen einer Krankheit bei einem Subjekt, oder
(ii) Diagnose oder Überwachung der Nierenfunktion bei einem Subjekt, oder
(iii) Diagnose einer Nierenkrankheit oder von Nierenkomplikationen vor dem Einsetzen der Nierendegeneration bei einem Subjekt, oder
(iv) Vorhersage oder Überwachung des Risikos eines unerwünschten Ereignisses bei einem Subjekt mit Nierenkrankheit, wobei das unerwünschte Ereignis ausgewählt ist aus der Gruppe, umfassend eine Verschlechterung von Nierenfunktionsstörung, einschließlich Nierenversagen, Verlust der Nierenfunktion, Entwicklung eines verwandten komorbiden Zustands, Nierenkrankheit im Endstadium oder Tod, oder
(v) Vorhersage oder Überwachung des Erfolgs einer Therapie oder Intervention bei einem Subjekt mit Nierenkrankheit,

Umschließen eines oder mehrerer Behälter, umfassend:

(a) ein Substrat, das eine Oberfläche und einen ersten proUGN-Antikörper, der an der Oberfläche haftet, aufweist;
(b) einen zweiten proUGN-Antikörper, umfassend einen detektierbaren Marker;
wobei mindestens einer von dem ersten und dem zweiten proUGN-Antikörper monoklonale Antikörper sind, die spezifisch an menschliches proUGN oder ein Fragment oder eine posttranslationale Modifikation davon binden, wobei das menschliche proUGN oder das Fragment oder die posttranslationale Modifikation davon von einer menschlichen Nierenzelle sezerniert wird; und
(c) Anweisungen zum Durchführen einer oder mehrerer der in einem der Punkte (i) bis (v) genannten diagnostischen und/oder prognostischen Korrelationen.

16. Kit nach Anspruch 15, wobei der erste und der zweite proUGN-Antikörper monoklonale Antikörper sind.

17. Kit nach Anspruch 15 oder 16, wobei der erste und der zweite proUGN-Antikörper in der Lage sind, proUGN zu binden, das die in SEQ ID NO: 1 dargelegte Sequenz aufweist.

18. Kit nach einem der Ansprüche 15 bis 17, wobei die Krankheit eine chronische Nierenkrankheit (CKD) ist.

**Revendications**

1. Procédé de

   (i) diagnostic d'une dysfonction ou d'une maladie rénale et/ou de complications rénales d'une maladie chez un sujet, ou
   (ii) diagnostic ou surveillance de la fonction rénale chez un sujet, ou
   (iii) diagnostic d'une maladie rénale ou de complications rénales avant la survenue d'une dégénérescence rénale chez un sujet, ou
   (iv) prédiction ou surveillance du risque d'un événement indésirable chez un sujet atteint d'une maladie rénale, dans lequel ledit événement indésirable est sélectionné dans le groupe comprenant une aggravation d'une dysfonction rénale incluant une insuffisance rénale, une perte de la fonction rénale, le développement d'une comorbidité associée, une insuffisance rénale terminale, ou le décès, ou
   (v) prédiction ou surveillance du succès d'une thérapie ou d'une intervention chez un sujet atteint d'une maladie rénale, le procédé comprenant :

   (a) la mesure d'un niveau ou d'une quantité de prouroguanyline (proUGN) dans un échantillon biologique,
   (b) la comparaison de la mesure avec une plage de valeurs de référence ou une valeur seuil,
   (c) la détermination du fait que la mesure de la proUGN est supérieure à la plage de valeurs de référence ou à la valeur seuil.

2. Le procédé selon la revendication 1, dans lequel la plage de valeurs de référence ou la valeur seuil est une plage normale de valeurs pour des sujets non atteints de maladie rénale.

3. Le procédé selon la revendication 1 ou 2, dans lequel la mesure du niveau ou de la quantité de proUGN dans l'échantillon biologique comprend la détermination du niveau de prouroguanyline (proUGN), ou de ses fragments ou modifications post-traductionnelles de celle-ci.

4. Le procédé selon la revendication 3, dans lequel la mesure du niveau ou de la quantité de proUGN dans l'échantillon biologique comprend l'utilisation d'au moins un agent de liaison spécifique à la prouroguanyline (proUGN), ou à des fragments ou à modifications post-traductionnelles de celle-ci.

5. Le procédé selon la revendication 4, dans lequel le au moins un agent de liaison est un anticorps, un fragment d'anticorps, ou une protéine de liaison de structure différente d'une Ig.

6. Le procédé selon la revendication 5, dans lequel le au moins un agent de liaison se lie à une région d'au moins 4 acides aminés de la prouroguanyline (proUGN) de pleine longueur, à un fragment N-terminal de la proUGN, ou à un fragment C-terminal de la proUGN.

7. Le procédé selon la revendication 6, dans lequel la prouroguanyline (proUGN) de pleine longueur possède la séquence

   **vyiqyqgfrvqlesmkklsdleaqwapsprlqaqsllpavchhpalpqdlqpvcasqeassif ktlrtianddcelcvnvactgcl** (SEQ ID NO: 1), le fragment N-terminal de la proUGN possède la séquence
   **vyiqyqgfrvqlesmkklsdleaqwapsprlqaqsllpavchhpalpqdlqpvcasqeassif ktlrtia** (SEQ ID NO: 2), et le fragment C-terminal de la proUGN possède la séquence **tianddcelcvnvactgcl** (SEQ ID NO: 3) et le fragment C-terminal de la proUGN possède la séquence **nddcelcvnvactgcl** (SEQ ID NO: 4).

8. Le procédé selon l'une quelconque des revendications précédentes, dans lequel l'étape (a) est une mesure quantitative de la prouroguanyline (proUGN) qui comprend un dosage immunologique sélectionné dans le groupe consistant en un dosage d'immunoabsorption enzymatique (ELISA), un dosage à flux latéral, un dosage immunologique par comptage (CIA), un dosage immunologique par chimiluminescence (CLIA), un dosage radioimmunologique (RIA), un dosage immunoradiométrique (IRMA) et un western blot, de préférence un ELISA.

9. Le procédé selon l'une quelconque des revendications précédentes, dans lequel l'échantillon biologique est sélectionné dans le groupe consistant en de l'urine, du sang, du sérum, du plasma, de la salive, du liquide céphalorachidien, du liquide amniotique, un lavage pulmonaire ou nasal, et du liquide gastrointestinal.

**10.** Le procédé selon la revendication 8, dans lequel l'ELISA comprend :

le transfert de l'échantillon biologique sur une plaque de capture comprenant un anticorps monoclonal anti-proUGN immobilisé ;
le retrait de l'échantillon biologique de la plaque de capture ;
l'ajout d'un deuxième anticorps monoclonal anti-proUGN à la plaque de capture, le deuxième anticorps monoclonal anti-proUGN étant conjugué à un marqueur détectable ; et
la détermination d'un niveau du marqueur détectable.

**11.** Le procédé selon l'une quelconque des revendications précédentes, dans lequel la plage de valeur de référence ou la valeur seuil correspond à 0,91 ng/ml de proUGN.

**12.** Le procédé selon la revendication 10, dans lequel le niveau du marqueur détectable supérieur à la plage de valeur de référence ou à la valeur seuil indique une maladie rénale chronique de stade 1, stade 2, stade 3, stade 4 ou stade 5.

**13.** Le procédé selon la revendication 8, dans lequel le test à flux latéral comprend la capture de la proUGN avec un anticorps monoclonal de capture, et la liaison d'un anticorps monoclonal de détection à la proUGN.

**14.** Le procédé selon l'une quelconque des revendications précédentes, dans lequel la maladie est la maladie rénale chronique (MRC).

**15.** Kit pour

(i) diagnostiquer une dysfonction ou une maladie rénale et/ou des complications rénales d'une maladie chez un sujet, ou
(ii) diagnostiquer ou surveiller la fonction rénale chez un sujet, ou
(iii) diagnostiquer une maladie rénale ou des complications rénales avant la survenue d'une dégénérescence rénale chez un sujet, ou
(iv) prédire ou surveiller le risque d'événement indésirable chez un sujet atteint d'une maladie rénale, dans lequel ledit événement indésirable est sélectionné dans le groupe comprenant une aggravation d'une dysfonction rénale incluant une insuffisance rénale, une perte de la fonction rénale, le développement d'une comorbidité associée, une insuffisance rénale terminale, ou le décès, ou
(v) prédire ou surveiller le succès d'une thérapie ou d'une intervention chez un sujet atteint d'une maladie rénale,

englobant un ou plusieurs contenants, comprenant :

(a) un substrat possédant une surface et un premier anticorps anti-proUGN adhérant à la surface ;
(b) un deuxième anticorps anti-proUGN comprenant un marqueur détectable ;
dans lequel au moins un des premier et deuxième anticorps anti-proUGN est un anticorps monoclonal qui se lie spécifiquement à la proUGN humaine, ou à un fragment ou à une modification post-traductionnelle de celle-ci, dans lequel la proUGN humaine, ou le fragment ou la modification post-traductionnelle de celle-ci, est sécrété(e) à partir d'une cellule rénale humaine ; et
(c) des instructions pour réaliser une ou plusieurs des corrélations diagnostiques et/ou pronostiques décrites dans l'un quelconque de (i) à (v).

**16.** Le kit selon la revendication 15, dans lequel les premier et deuxième anticorps anti-proUGN sont des anticorps monoclonaux.

**17.** Le kit selon la revendication 15 ou 16, dans lequel les premier et deuxième anticorps anti-proUGN sont capables de se lier à la proUGN possédant la séquence telle que décrite dans SEQ ID NO: 1.

**18.** Le kit selon l'une quelconque des revendications 15 à 17, dans lequel la maladie est la maladie rénale chronique (MRC).

FIG. 1

FIG. 2

ROC CURVE: CONTROLS VS ALL CKD

FIG. 3

ROC CURVE: "LATE CKD" VS "CONTROLS/EARLY CKD"

FIG. 4

ROC CURVE: "ESRD" VS "CONTROLS/EARLY/STAGE 4 CKD"

AUC: 0.9756

SENSITIVITY

1-SPECIFICITY, FALSE POSITIVE RATE

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080221022 A
- US 8173596 B
- US 5234933 A
- US 5326902 A
- US 5489670 A
- US 5879656 A
- US 6180082 B
- US 7522762 B
- US 20090181852 A1
- US 20110306125 A1
- US 8129191 B
- US 8361389 B
- US 9090698 B
- US 9708371 B
- US 9814752 B
- US 9845362 B
- US 10118946 B
- US 10232011 B
- WO 12143769 A
- WO 7977110 A
- WO 13118932 A
- WO 8602996 A
- WO 13945477 A
- WO 8815602 A

- WO 13128392 A
- WO 8993250 A
- WO 13061446 A
- WO 9057735 A
- WO 13125631 A
- WO 9128107 A
- WO 14041682 A
- WO 9232897 A
- WO 14738904 A
- WO 9417250 A
- WO 13793833 A
- WO 9689826 A
- WO 15664476 A
- WO 10156564 A
- WO 15478007 A
- WO 10269447 A
- WO 15034338 A
- WO 10274502 A
- WO 14421382 A
- WO 10444248 A
- WO 9325521 A
- WO 9706258 A
- WO 9720049 A

**Non-patent literature cited in the description**

- Robbins Basic Pathology. Elsevier, 2018
- Kidney Disease Improving Global Outcomes (KDI-GO). *Kidney International Suppl*, 2013, vol. 3, 5-14
- Kidney Disease Outcomes Quality Initiative (KDOQI). *American J. Kidney Dis.*, 2014, vol. 63, 713-735
- **KAAR G** ; **DIEPLINGER B** ; **GABRIEL C** ; **HALTMAYER M** ; **MUELLER T**. Proguanylin and prouroguanylin--assay evaluation and clinical analyte characterization. *Clin Chim Acta.*, 20 November 2011, vol. 412 (23-24), 2277-83
- Fundamental Immunology. Raven Press, 1993
- **WILSON**. *J. Immunol. Methods*, 1994, vol. 175, 267-273
- **YARMUSH**. *J. Biochem. Biophys. Methods*, 1992, vol. 25, 85-97
- **WARD et al.** *Nature*, 1989, vol. 341, 544-546
- Harrison's Principles of Internal Medicine. McGraw Hill, 1741-1830

- Current Medical Diagnosis & Treatment. McGraw Hill, 2008, 785-815
- **AYDIN et al.** *J. Vis. Exp.*, 2012, vol. 65, e4041
- **LEVI et al.** *Ann Intern Med*, 2009, vol. 150 (9), 604-612
- *J American Stat Assoc*, 1952, vol. 47, 583-621
- **STERN et al.** Essential Medical Statistics. Oxford Blackwell Science, 2003, 48
- Merck Manual of Diagnosis and Therapy. Merck Research Laboratories, Whitehouse Station, 1999
- 2018 USRDS Annual Data Report: Epidemiology of Kidney Disease in the United States. N. National Institutes of Health, 2018
- USRDS, 2014 annual data report: An overview of the epidemiology of kidney disease in the United States. 2014
- KDOQI Clinical Practice Guideline for Diabetes and CKD: 2012 Update. *Am J Kidney Dis*, 2012, vol. 60 (5), 850-86

- **SATCHIDANAND, N. et al.** Positive Predictive Value of a Single Assessment of Estimated GFR in the Diagnosis of Chronic Kidney Disease. *South Med J*, 2016, vol. 109 (6), 351-5
- KDIGO 2012 clinical practice guideline for the evaluation and management of chronic kidney disease. *Kidney International Suppl*, 2013, vol. 3 (1), 1-150
- **MCTAGGART, M.P. et al.** The diagnostic accuracy of a urine albumin-creatinine ratio point-of-care test for detection of albuminuria in primary care. *Am J Kidney Di*, 2012, vol. 60 (5), 787-94
- **VAN STRALEN, K.J. et al.** Diagnostic methods I: sensitivity, specificity, and other measures of accuracy. *Kidney Int*, 2009, vol. 75 (12), 1257-1263
- **WAIKAR, S.S. et al.** Biological Variability of Estimated GFR and Albuminuria in CKD. *Am J Kidney Dis*, 2018, vol. 72 (4), 538-546
- **GAITONDE, D.Y.** ; **D.L. COOK** ; **I.M. RIVERA**. Chronic Kidney Disease: Detection and Evaluation. *Am Fam Physician*, 2017, vol. 96 (12), 776-783
- **FOX, C.H. et al.** A quick guide to evidence-based chronic kidney disease care for the primary care physician. *Postgraduate Medicine*, 2008, vol. 120 (2), 1-6
- **RAVIZZA, S. et al.** Predicting the early risk of chronic kidney disease in patients with diabetes using real-world data. *Nat Med*, 2019, vol. 25 (1), 57-59
- **NARESH, C.N. et al.** Day-to-day variability in spot urine albumin-creatinine ratio. *Am J Kidney Dis*, 2013, vol. 62 (6), 1095-101
- **RYAN, T.P. et al.** Chronic kidney disease prevalence and rate of diagnosis. *Am J Med*, 2007, vol. 120 (11), 981-6
- **INKER, L.A. et al.** KDOQI US commentary on the 2012 KDIGO clinical practice guideline for the evaluation and management of CKD. *Am J Kidney Dis*, 2014, vol. 63 (5), 713-35
- **CORESH, J. et al.** Current CKD Definition Takes into Account Both Relative and Absolute Risk. *J Am Soc Nephrol*, 2019
- Chronic Kidney Disease in the United States 2019. Centers for Disease Control and Prevention. US Department of Health and Human Services, 2019, vol. 1, 1
- **OKUSA, M.D.** A hidden epidemic: More than 850 million suffer from kidney diseases worldwide, organizations report. *Kidney News*, 2019, vol. 5 (5), 18-19
- **LEVEY, A.S. et al.** National Kidney Foundation practice guidelines for chronic kidney disease: Evaluation, classification, and stratification. *Ann Intern Med*, 2003, vol. 139 (2), 137-47
- National Kidney Foundation: Kidney Disease. National Kidney Foundation: Kidney disease, 2008
- **NARVA, A.S.** The National Kidney Disease Education Program and other related efforts in the United States. *Scand J Clin Lab Invest Suppl*, 2008, vol. 241, 12-5
- National Health and Examination Survey. National Center for Health Statistics. 10 January 2020
- Healthy People 2020. Healthy People provides science-based, 10-year national objectives for improving the health of all Americans. Healthy People.Gov, 21 July 2011
- Healthy People 2010 Objectives: Chronic Kidney Disease. Reduce the incidence, morbidity, mortality and health care costs of chronic kidney disease. HealthPeople.Gov, 24 February 2010
- **PERGOLA, P.E. et al.** Effect of bardoxolone methyl on kidney function in patients with T2D and Stage 3b-4 CKD. *Am J Nephrol*, 2011, vol. 33 (5), 469-76
- **PERGOLA, P.E. et al.** Bardoxolone Methyl and Kidney Function in CKD with Type 2 Diabetes. *N Engl J Med*, 2011, vol. 365 (4), 327-336
- **FORTE, L.R., JR.** Uroguanylin: Physiological role as a natriuretic hormone. *J Am Soc Nephrol*, 2005, vol. 16 (2), 291-2
- **FORTE, L.R.** ; **X. FAN** ; **F.K. HAMRA**. Salt and water homeostasis: Uroguanylin is a circulating peptide hormone with natriuretic activity. *Am J Kidney Dis*, 1996, vol. 28 (2), 296-304
- **FORTE, L.R. et al.** Guanylin peptides: renal actions mediated by cyclic GMP. *Am J Physiol Renal Physiol*, 2000, vol. 278 (2), F180-91
- **FORTE, L.R., JR.** Uroguanylin and guanylin peptides: pharmacology and experimental therapeutics. *Pharmacol Ther*, 2004, vol. 104 (2), 137-62
- **KINOSHITA, H. et al.** Urine and plasma levels of uroguanylin and its molecular forms in renal diseases. *Kidney Internationa*, 1997, vol. 52 (4), 1028-1034
- **HESS, R. et al.** GCAP-II: isolation and characterization of the circulating form of human uroguanylin. *FEBS Lett*, 1995, vol. 374 (1), 34-8
- **KLODT, J. et al.** Synthesis, biological activity and isomerism of guanylate cyclase C-activating peptides guanylin and uroguanylin. *J Pept Res*, 1997, vol. 50 (3), 222-30
- **LI, Z. et al.** Purification, cDNA sequence, and tissue distribution of rat uroguanylin. *Regul Pept*, 1997, vol. 68 (1), 45-56
- **PERKINS, A.** ; **M.F. GOY** ; **Z. LI**. Uroguanylin is expressed by enterochromaffin cells in the rat gastrointestinal tract. *Gastroenterology*, 1997, vol. 113 (3), 1007-14
- **NAKAZATO, M. et al.** Tissue distribution, cellular source, and structural analysis of rat immunoreactive uroguanylin. *Endocrinology*, 1998, vol. 139 (12), 5247-54

- **NAKAZATO, M.** Guanylin family: new intestinal peptides regulating electrolyte and water homeostasis. *J Gastroenterol*, 2001, vol. 36 (4), 219-25
- **SINDIC, A.** Current understanding of guanylin peptides actions. *ISRN Nephrol*, 2013, vol. 2013, 813648
- **SINDIC, A. et al.** Guanylin and uroguanylin regulate electrolyte transport in isolated human cortical collecting ducts. *Kidney Int*, 2005, vol. 67 (4), 1420-7
- **CARRITHERS, S.L. et al.** Site-specific effects of dietary salt intake on guanylin and uroguanylin mRNA expression in rat intestine. *Regul Pept*, 2002, vol. 107 (1-3), 87-95
- **CARRITHERS, S.L. et al.** Guanylyl cyclase-C (GC-C) receptor mRNA distribution in the rat nephron. *Journal of Investigative Medicine*, 2000, vol. 48 (2), 20
- **MIYAZATO, M. et al.** Uroguanylin gene expression in the alimentary tract and extra-gastrointestinal tissues. *FEBS Letters*, 1996, vol. 398 (2-3), 170-174
- **KULAKSIZ, H.** ; **Y. CETIN**. Uroguanylin and guanylate cyclase C in the human pancreas: expression and mutuality of ligand/receptor localization as indicators of intercellular paracrine signaling pathways. *Journal of Endocrinology*, 2001, vol. 170 (1), 267-275
- **MUELLER, T.** ; **B. DIEPLINGER**. The guanylin peptide family and the proposed gastrointestinal-renal natriuretic signaling axis. *Kidney Int*, 2012, vol. 82 (12), 1253-5
- **VALENTINO, M.A. et al.** A uroguanylin-GUCY2C endocrine axis regulates feeding in mice. *J Clin Invest*, 2011, vol. 121 (9), 3578-88
- **MOSS, N.G. et al.** Uroguanylin, an intestinal natriuretic peptide, is delivered to the kidney as an unprocessed propeptide. *Endocrinology*, 2008, vol. 149 (9), 4486-98
- **QIAN, X. et al.** Circulating prouroguanylin is processed to its active natriuretic form exclusively within the renal tubules. *Endocrinology*, 2008, vol. 149 (9), 4499-509
- **QIAN, X. et al.** The rat kidney contains high levels of prouroguanylin (the uroguanylin precursor) but does not express GC-C (the enteric uroguanylin receptor). *Am J Physiol Renal Physiol*, 2011, vol. 300 (2), F561-73
- **CARRITHERS, S.L. et al.** Natriuretic and kaliuretic properties of guanylin and uroguanylin in vivo. *Journal of Investigative Medicine*, 1999, vol. 47 (4), 183A-183A
- **CARRITHERS, S.L. et al.** Renal effects of uroguanylin and guanylin in vivo. *Brazilian Journal of Medical and Biological Research*, 1999, vol. 32 (11), 1337-1344
- **FORTE, L.R. et al.** Mechanisms of guanylin action via cyclic GMP in the kidney. *Annual Review of Physiology*, 2000, vol. 62, 673-695
- **CARRITHERS, S.L. et al.** Guanylin and uroguanylin induce natriuresis in mice lacking guanylyl cyclase-C receptor. *Kidney Int*, 2004, vol. 65 (1), 40-53
- **CARRITHERS, S.L. et al.** Guanylyl cyclase-C receptor mRNA distribution along the rat nephron. *Regul Pept*, 2000, vol. 95 (1-3), 65-74
- **LEVEY, A.S. et al.** Definition and classification of chronic kidney disease: a position statement from Kidney Disease: Improving Global Outcomes (KDIGO). *Kidney Int*, 2005, vol. 67 (6), 2089-100
- **MURPHY, D. et al.** Trends in prevalence of chronic kidney disease in the United States. *Ann Intern Med*, 2016, vol. 165 (7), 473-481
- **HUI, X. et al.** CKD and cardiovascular disease in the Atherosclerosis Risk in Communities (ARIC) study: interactions with age, sex, and race. *Am J Kidney Dis*, 2013, vol. 62 (4), 691-702
- **YANG, M. et al.** Complications of progression of CKD. *Adv Chronic Kidney Dis*, 2011, vol. 18 (6), 400-405
- **ANDRASSY, K.M.** Commentary of 'KDIGO 2012 clinical practice guideline for the evaluation and management of chronic kidney disease.. *Kidney Int'1*, 2013, vol. 84 (3), 622-623
- **QASEEM, A. et al.** Screening, monitoring, and treatment of stage 1 to 3 chronic kidney disease: A clinical practice guideline from the American College of Physicians. *Ann Intern Med*, 2013, vol. 159 (12), 835-47
- **TALER, S.J. et al.** KDOQI US commentary on the 2012 KDIGO clinical practice guideline for management of blood pressure in CKD. *Am J Kidney Dis*, 2013, vol. 62 (2), 201-13
- Clinical practice guidelines for chronic kidney disease: Evaluation, classification, and stratification. *Am J Kidney Dis*, 2002, vol. 39, S1-S266
- KDOQI Clinical practice Guideline for Diabetes and CKD: 2012 Update. *Am J Kidney Dis*, 2012, vol. 60 (5), 850-886
- KDIGO clinical practice guideline for lipid management in chronic kidney disease. *Kidney Int'l Suppl*, 2013, vol. 3 (3), 159-305
- **PALEVSKY, P.M. et al.** KDOQI US commentary on the 2012 KDIGO clinical practice guideline for acute kidney injury. *Am J Kidney Dis*, 2013, vol. 61 (5), 649-72
- **MEFFORD, M.T. et al.** Trends in Statin Use Among US Adults With Chronic Kidney Disease, 1999-2014. *J Am Heart Assoc*, 2019, vol. 8 (2), e010640
- KDIGO 2017: Clinical practice guideline update for the diagnosis, evaluation, prevention, and treatment of chronic kidney disease - Mineral and Bone Disorder (CKD-MBD). *Kidneys*, 2017, vol. 6 (3), 149-154